# EUROPEAN PATENT SPECIFICATION

(11) **EP 1 439 885 B1**
(45) Date of publication and mention of the grant of the patent: **30.08.2006**
(21) Application number: 02772277.6
(22) Date of filing: 06.09.2002
(51) Int. Cl.: A61P 35/00, C07C 69/16, C07D 233/54, C07D 277/24, C07D 263/32, C07D 213/30, A61K 31/415, A61K 31/44, A61K 31/42, A61K 31/425

(54) **SIMPLIFIED SARCODICTYN DERIVATIVES AS ANTI-TUMOR AGENTS**
SIMPLIFIZIERTE SARCODICTYN-DERIVATE ALS ANTI-TUMOR-MITTEL
DERIVES DE SARCODICTYNE A STRUCTURE CHIMIQUE SIMPLIFIEE, UTILISES COMME AGENTS ANTITUMORAUX

(30) Priority: 14.09.2001 GB 0122257
(43) Date of publication of application: 28.07.2004
(73) Proprietor: Pfizer Italia S.r.l., 04010 Latina (IT); Universita Degli Studi Di Milano, 20122 Milano (IT)
(72) Inventor: MONGELLI, Nicola, I-20137 Milano (IT); MENICHINCHERI, Maria, I-20124 Milano (IT); CIOMEI, Marina, I-20094 Corsico (IT); GENNARI, Cesare, I-20122 Milano (IT); TELSER, Joachim, 42105 Wuppertal (DE); BEUMER, Raphael, 31840 Hessisch Oldendorf (DE)
(74) Representative: Modiano, Micaela Nadia
(86) International application number: PCT/EP2002/010038
(87) International publication number: WO 2003/024529

(56) References cited:
- US-A- 5 869 514
- TELSER J ET AL: "Synthesis of a simplified sarcodictyin analogue which retains microtubule stabilising properties" TETRAHEDRON LETTERS, ELSEVIER SCIENCE PUBLISHERS, AMSTERDAM, NL, vol. 42, no. 52, 24 December 2001 (2001-12-24), pages 9187-9190, XP004328109 ISSN: 0040-4039

## Description

The present invention relates to new anti-tumor agents, to a process for their preparation, to pharmaceutical compositions containing them, and to the use of such compounds in the prevention, control and treatment of cancer. Sarcodictyins A and B were isolated in 1987 by Pietra *et al.* from the Mediterranean stoloniferan coral *Sarcodictyon roseum,* while their anti-tumor activity was recognised about a decade later, and their paclitaxel-like mechanism of action uncovered (Ciomei, M. et al. *Proc. Amer. Ass. Canc. Res.* **1997,** *38,* 5 and WO 96 36 335-A1)

In the meantime, the diterpene glycoside eleutherobin was reported by Fenical *et al.* from an *Eleutherobia* species of australian soft coral, accompanied by disclosure of its potent cytotoxicity. Two years later, in 1997, it was shown that eleutherobin, similarly to sarcodictyins, acted by mitotic arrest through induced tubulin polymerization.

Now, there is a strong need for more simplified molecules, which nevertheless maintain the useful properties referred to above characterizing the natural substances.

The present invention relates to a new class of simplified sarcodictyins. In particular, the present invention provides a compound which is a sarcodictyin derivative of formula (I) 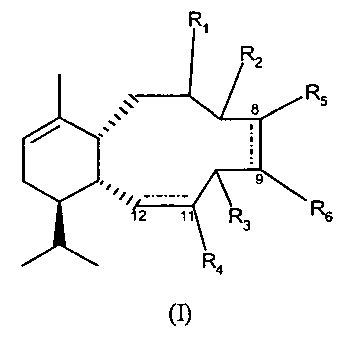 wherein:
----- at positions 8-9 and 11-12 independently represents a single or double bond,
-R₁ represents oxygen (=O), or a residue -OR₇, wherein R₇ represents hydrogen, linear or branched C₁-C₇ alkanoyl, benzoyl, C₁-C₁₀ alkyl, C₂-C₁₀ alkenyl or a residue of the formula 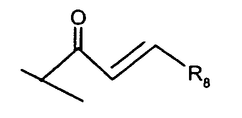
wherein R₈ is an aryl or heterocyclyl;
one of -R₂ and -R₃ represents hydrogen and the other one is chosen from the group consisting of hydrogen, oxygen (=O) and a residue -OR₉, wherein R₉ represents hydrogen, C₁-C₆ alkanoyl or benzoyl;
when ----- at position 11-12 represents a single bond, then -R₄ represents oxygen (=O), methylene (=CH₂), =CHCOOR₁₀, wherein R₁₀represents C₁-C₁₀ alkyl or aryl; =H(OCH₃), or a residue of formula
-OR₉, wherein R₉ is as defined above;
-CH₂OR₁₁ wherein R₁₁ represents hydrogen or a sugar residue,
-COR₁₂ wherein R₁₂ represents hydrogen, -OH or -OR₁₀, wherein R₁₀ is as defined above; or when ----- at position 11-12 represents a double bond, then -R₄ represents a residue of formula -CH₂OR₁₁ or -COR₁₂ as defined above;
- R₅ and -R₆ are both hydrogen or, when ----- at position 8-9 represents a single bond, taken together with the carbon atoms to which they are attached form a cyclopropane ring; or a pharmaceutically acceptable salt thereof

As used herein the term "C₁-C₁₀ alkyl" refers to a straight or branched chain alkyl moiety having from 1 to 10 carbon atoms, including for example, methyl, ethyl, propyl, isopropyl, n-butyl, i-butyl, sec-butyl, tert-butyl, n-pentyl, isopentyl, n-hexyl, n-heptyl and nmoctyl.

The term "C₂-C₁₀ alkenyl" as used herein refers to a straight or branched chain alkenyl moiety having from 2 to 10 carbon atoms and having in addition one double bond of either E or Z stereochemistry where applicable. Examples of alkenyl groups include: vinyl, allyl, metallyl, butenyl and crotyl. The term "aryl" as used herein refers to a monocyclic or bicyclic aromatic hydrocarbon group of 6 to 10 carbon atoms, such as phenyl, naphthyl, indanyl; furthermore, "aryl" as used herein may refer to a diphenyl group (-C₆H₄-C₆H₅). The term "C₁-C₇ alkanoyl" refers to acyl residues such as formyl, acetyl, pivaloyl (PIV), and pentanoyl groups.

The term "heterocyclyl" as used herein refers to a 3- to 7-membered, substituted or unsubstituted, saturated or unsaturated heterocyclyl ring, containing at least one heteroatom selected from O, S and N, any ring carbon may be oxidized as a carbonyl, and wherein said heterocyclyl ring may be optionally fused to a second 5- or 6-membered, saturated or unsaturated heterocyclyl ring, or to a C₃ -C₇ cycloalkyl ring, or to a benzene or naphthalene ring. Examples of heterocyclyl groups are pyrrolyl, pyrrolidinyl, pyrazolyl, imidazolyl, triazolyl, tetrazolyl, oxazolyl, isoxazolyl, thiazolyl, isothiazolyl, thiadiazolyl, thienyl, tetrahydrothienyl, furyl, tetrahydrofuryl, aziridinyl, oxiranyl, azetidinyl, succinimido, pyridyl, piperidinyl, pyrazinyl, piperazinyl, pyridazinyl, hexahydropyridazinyl, pyrimidinyl, pyranyl, tetrahydropyranyl, benzothienyl, benzothiazolyl, benzoxazolyl, isobenzofuranyl, benzofuranyl, benzimidazolyl, indazolyl, chromenyl, indolyl, oxindolyl, phthalimido, 1-oxo-2-isoindolyl, quinolyl, isoquinolyl, tetrahydroisoquinolyl, indolizinyl, isoindolyl, 2-oxoisoindolyl, 1,2-(methylenedioxy)phenyl, quinuclidinyl, hydantoinyl, saccarinyl, cinnolinyl, purinyl, morpholinyl, thiomorpholinyl, dioxanyl, dithianyl and azepinyl.

Most preferred heterocyclyl groups are N-methyl-imidazolyl, 2-methyl-thiazolyl, 2-methyl-oxazolyl and pyridyl group. Preferably, when OR₁₁ is a sugar residue, it has the formula 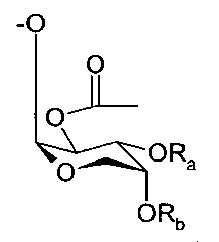 wherein Rₐ and R_{b} independently represent hydrogen, a hydroxy protecting group, or C₁-C₆ alkanoyl.

Substituents which may be present in the aryl or heterocyclyl groups in any of the above definitions of R₁- R₁₀ include the following:
- halo (i.e., fluoro, bromo, chloro or iodo);
- hydroxy;
- nitro;
- azido;
- mercapto (i.e., -SH), and acetyl or phenylacetyl esters thereof (i.e., -SCOCH₃ and - SCOCH₂C₆H₅);
- amino (i.e., -NH₂ or -NHR^{I} or -NR^{I}R^{II}, wherein R^{I} and R^{II}, which are the same or different, are straight or branched C₁-C₆ alkyl, phenyl, biphenyl (i.e., -C₆H₄-C₆H₅), or benzyl groups, optionally substituted by hydroxy, methoxy, methyl, amino, methylamino, dimethylamino, chloro or fluoro; or R^{I} and R^{II} taken together with the nitrogen atom to which they are attached form a heterocyclic ring such as morpholino, pyrrolidino, piperidino, pyperazino or N-methylpyperazino;
- guanidino, i.e., -NHC(=NH)NH₂;
- formyl (i.e. -CHO);
- cyano;
- carboxy (i.e. -COOH), or esters thereof (i.e., -COOR^{I}), or amides thereof (i.e., -CONH₂, - CONHR' or -CONHR^{I}R^{II}), wherein R^{I} and R^{II} are as defined above, and including morpholino-amides, pyrrolidino-amides, and carboxymethylamides -CONHCH₂COOH;
- sulfo (i.e., -SO₃H);
- acyl, i.e., -C(O)R^{I}, wherein R^{I} is as defined above, including monofluoroacetyl, difluoroacetyl, trifluoroacetyl;
- carbamoyloxy (i.e., -OCONH₂) and N-methylcarbamoyloxy;
- acyloxy, i.e., -OC(O)R^{I} wherein R^{I} is as defined above, or formyloxy;
- acylamino, i.e., -NHC(O)R^{I}, or -NHC(O)OR^{I} , wherein R^{I} is as defined above or is a group -(CH₂)ₜ COOH where t is 1,2 or 3;
- ureido, i.e., -NH(CO)NH₂, -NH(CO)NHR^{I}, -NH(CO)NR^{I}R^{II}, wherein R^{I} and R^{II} are as defined above, including -NH(CO)-(4-morpholino), -NH(CO)-(1-pyrrolidino), -NH(CO)-(1-piperazino), -NH(CO)-(4-methyl-1-piperazino);
- sulfonamido, i.e., -NHSO₂R^{I} wherein R^{I} is as defined above;
- a group -(CH₂)ₜCOOH, and esters and amides thereof, i.e., -(CH₂)ₜCOOR^{I} and - (CH₂)ₜCONH₂, -(CH₂)ₜCONHR^{I}, -(CH₂)ₜCONR^{I}R^{II}, wherein t, R^{I} and R^{II} are as defined above;
- a group -NH(SO₂)NH₂, -NH(SO₂)NHR^{I}, -NH(SO₂)NR^{I}R^{II}, wherein R^{I} and R^{II} are as defined above, including -NH(SO₂)-(4-morpholino), -NH(SO₂)-(1-pyrrolidino), -NH(SO₂)-(1-piperazino), -NH(SO₂)-(4-methyl-1-piperazino);
- a group -OC(O)OR^{I}, wherein R^{I} is as defined above;
- a group -OR^{I}, wherein R^{I} is as defined above, including -OCH₂COOH;
- a group -SR^{I}, wherein R^{I} is as defined above, including -SCH₂COOH;
- a group -S(O)R^{I}, wherein R^{I} is as defined above;
- a group -S(O₂)R^{I}, wherein R^{I} is as defined above;
- a group -SO₂NH₂, -SO₂NHR^{I}, or - SO₂NR^{I}R^{II}, wherein R^{I} and R^{II} are as defined above;
- C₁ -C₆ alkyl or C₂ -C₆ alkenyl;
- C₃ -C₇ cycloalkyl;
- substituted methyl selected from chloromethyl, fluoromethyl, difluoromethyl, trifluoromethyl, aminomethyl, N,N-dimethylaminomethyl, azidomethyl, cyanomethyl, carboxymethyl, sulfomethyl, carbamoylmethyl, carbamoyloxymethyl, hydroxymethyl, methoxycarbonylmethyl, ethoxycarbonylmethyl, tert-butoxycarbonylmethyl and guanidinomethyl.

When present, carboxy, hydroxy, mercapto and amino groups may be either free or in a protected form. Protected forms of said groups are any of those generally known in the art. Preferably, carboxy groups are protected as esters thereof, in particular methyl, ethyl, tert-butyl, benzyl, and 4-nitrobenzyl esters. Preferably, hydroxy groups are protected as silyl-ethers, ethers or esters thereof, in particular trimethyl silyl, tert-butyldiphenyl silyl, triethyl silyl, triisopropyl silyl or tert-butyldimethylsilyl ethers, methoxymethyl ethers, tetrahydropyranyl ethers, benzyl ethers, acetates or benzoates. Preferably, mercapto groups are protected as thioethers or thioesters, in particular tert-butyl thioethers, thioacetates or thiobenzoates. Preferably, amino groups are protected as carbamates, e.g. tert-butoxycarbonyl derivatives, or as amides, e.g. acetamides and benzamides.

As stated above, the present invention provides the salts of those compounds of formula (I) that have salt-forming groups, especially the salts of the compounds having a carboxylic group or the salts of the compounds having a basic group, especially an amino. The salts are especially physiologically tolerable salts, for example alkali metal and alkaline earth metal salts (e.g. sodium, potassium, lithium, calcium and magnesium salts), ammonium salts and salts with an appropriate organic amine or amino acid (e.g. arginine, procaine salts), and the addition salts formed with suitable inorganic acids (e.g. hydrochlorides, hydrobromides, sulfates, phosphates) or carboxylic and sulfonic organic acids (e.g. acetates, trifluoroacetates, citrates, succinates, malonates, lactates, tartrates, fumarates, maleates, methanesulfonates, *p*-toluenesulfonates).

Furthermore, hydrates, solvates of compounds of formula (I), and physiologically hydrolyzable derivatives (i.e., prodrugs) of compounds of formula (I) are included within the scope of the present invention.

It is to be noted that the R₁, R₂, R₃ and R₄ substituents may be above or under the plane, so that the present invention encompasses all the possible stereoisomers (e.g. diastereoisomers, epimers, geometrical isomers) of the compounds of formula (I), as well as their racemic or optically active mixtures related to these substituents.

In the preferred configuration, the substituent R₁ is under the plane:

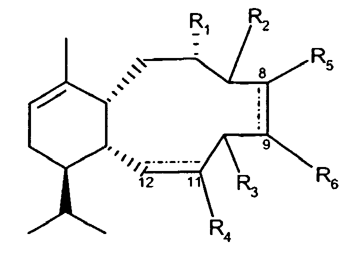

A preferred sarcodictyin derivative of the present invention has the following formula (Ia) 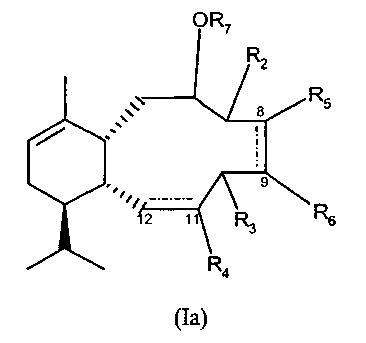 wherein:
----- at positions 8-9 and 11-12 independently represents a single or double bond,
R₇ represents a residue of the formula 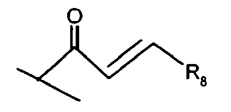
wherein R₈ is N-methyl imidazolyl, phenyl, methyl-thiazolyl, methyl-oxazolyl or pyridyl group;
one of -R₂ and -R₃ represents hydrogen and the other one is hydrogen or oxygen (=O), hydroxy, acetoxy ;
when ----- at position 11-12 represents a single bond, then -R₄ represents oxygen (=O), methylene (=CH₂), =CHCOOR₁₀, wherein R₁₀ represents methyl or ethyl, =CH(OCH₃), - CHO; hydroxy, acetoxy, pivaloyloxy group or -CH₂OR₁₁ wherein R₁₁ represents hydrogen or a sugar residue having the formula 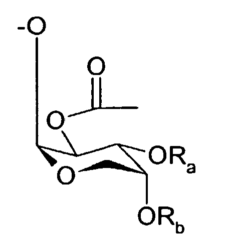
wherein Rₐ and R_{b} independently represent hydrogen, a hydroxy protecting group, or C₁-C₆ alkanoyl,
   or
when ----- at position 11-12 represents a double bond, then -R₄ represents a residue of formula -CO₂C₂H₅; and
- R₅ and -R₆ are both hydrogen atoms or, when ----- at position 8-9 represents a single bond, taken together with the carbon atoms to which they are attached form a cyclopropane ring.
The present invention also provides a process for preparing a compound of the invention as defined above, which process comprises:
cyclizing a compound of formula II 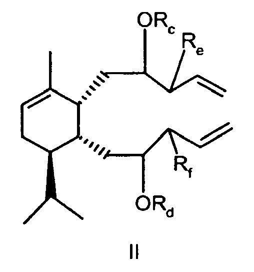
wherein R_{c} represents hydrogen, a silyl protecting group, C₁-C₆ alkanoyl or benzoyl or , taken together with Rₑ, forms an acetonide ring; R_{d} represents hydrogen, C₁-C₆ alkanoyl, or benzoyl, or , taken together with R_{f}, forms an acetonide ring; either Rₑ represents H and R_{f} either represents OH or is linked to the adjacent OR_{d} substituent as defined above, or R_{f} represents H and Rₑ either represents OH or is linked to the adjacent OR_{c} substituent as defined above;
and, if desired, converting the resulting sarcodictyin derivative of formula I', 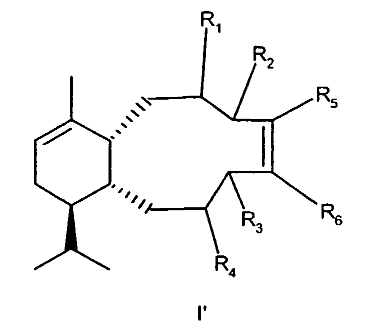
wherein R₁ is OR_{c}, R₂ is Rₑ, R₃ is R_{f}, R₄ is OR_{d}, in which R_{c}, R_{d}, Rₑ and R_{f} are as defined above and R₅ and R₆ are hydrogen atoms, into another sarcodicytin derivative of formula I as defined above; and/or if desired, converting a sarcodictyin derivative of formula I' or I into a pharmaceutically acceptable salt therof; and/or, if desired converting a pharmaceutically acceptable salt of a sarcodictyin derivative of formula I or I' into the corresponding free compound.

Preferably, R_{c} represents a silyl protecting group, more preferably a t-butyldiphenylsilyl group. The cyclization to give the compound of formula I' as single Z isomer can be performed through the Ring Closing Metathesis (RCM) reaction. In particular, the RCM reaction is carried out in the presence of an appropriate catalyst, more preferably a Nolan and Grubb's catalyst, described for example in J.Am.Chem.Soc., 1999, 121, 2674 and in Org. Lett., 1999, 1, 953. The conversion of a compound of formula I' or I into another different final compound of formula I may be carried out in several ways, depending on the meanings of the substituents and the presence of the unsaturated bonds in the ring. Such conversions follow conventional procedures which are known in the art.

For example, a compound of formula I wherein -R₁ represents a residue of the formula 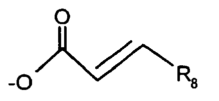 wherein R₈ is as defined above, can be obtained by condensing a corresponding compound of the formula I or I' wherein -R₁ represents hydroxy group with an activated form of the appropriate derivative of formula 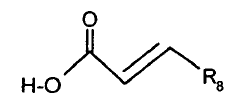 wherein R₈ is as above defined.

A compound of formula I wherein -R₂, -R₃ or -R₄ represents an oxygen atom =O can be obtained from a corresponding compound of formula I or I' as defined above wherein -R₂, -R₃ or -R₄ represents a hydroxy group by means of oxidation, for example with Dess-Martin periodinane, PDC or PCC or under Swem oxidation conditions (DMSO/oxalyl chloride), provided that the other hydroxy groups in the molecule, if any, are protected. A compound of formula I wherein -R₄ represents an oxygen atom =O can be conveniently converted into a corresponding compound of formula I wherein -R₄ represents methylene (=CH₂), =CHCOOR₁₀ wherein R₁₀ is as defined above, or =CH(OCH₃) by reaction with a suitable Wittig reagent, such as for example, respectively, Ph₃P=CH₂, Ph₃P=CHCOOR₁₀, wherein R₁₀ is as defined above and Ph₃P=CH(OCH₃). A compound of formula I wherein -R₄ represents =CH(OCH₃) can be then converted by acidic hydrolysis into a corresponding compound of formula I wherein -R₄ represents -CHO, which in turn may be either reacted with a reducing agent to give a compound of formula I wherein -R₄ represents -CH₂OH, or oxidised with a suitable reagent such as NaClO₂ to give a compound of formula I wherein -R₄ represents - COOH. A compound of formula I wherein -R₄ represents an oxygen atom =O can also be converted into a compound of formula I wherein -R₄ represents a -COOR₁₀ group wherein R₁₀ is as defined above and the bond at position 11-12 is double by treatment with triflic anhydride in the presence of a base followed by reaction of the resultant enol-triflate with CO and R₁₀-OH wherein R₁₀ is as defined above in the presence of Palladium catalyst and a base such as triethylamine according to known procedures as those described in J.Chem.Soc.Perkin Trans. I, 1991 (5), 969-979. Such compounds of formula I wherein -R₄ represents a -COOH group and the bond at position 11-12 is double can be converted by selective reduction into the corresponding 11-12 unsaturated compounds of formula I wherein -R₄ represents a -CH₂OH group, for example by treatment with ClCOOEt/NaBH₄

A compound of formula I' or I wherein the bond at position 8-9 is double may be converted into the corresponding compound of formula I with a single bond at the 8-9 position and wherein R₅ and R₆ are hydrogen atoms by hydrogenation, such as by treatment with H₂/(Ph₃P)₃RhCl (Ireland, R. E.; Bey, P. Org. Synth. 1973, 53, 63; Osborn, J. A.; Wilkinson, G. Inorg. Synth. 1977, 28, 77) or with diimide (NH=NH, Org.React. 1991, 40, 91); or into the corresponding compounds of formula I with a single bond at the 8-9 position wherein R₅ and R₆ taken together with the carbon atoms to which they are attached, form a cyclopropane ring by treatment with a suitable reactant such as a zinc carbenoid (J.Am.Chem.Soc. 2001, 123, 8139-8140).

A sarcodictyin derivative may be converting into a pharmaceutically acceptable salt thereof by salification, using conventional techniques. Suitable salts include those mentioned above. A compound of the formula II may be prepared as described in any one of the following scheme, in which R_{c} and R_{d} have the meanings above defined:

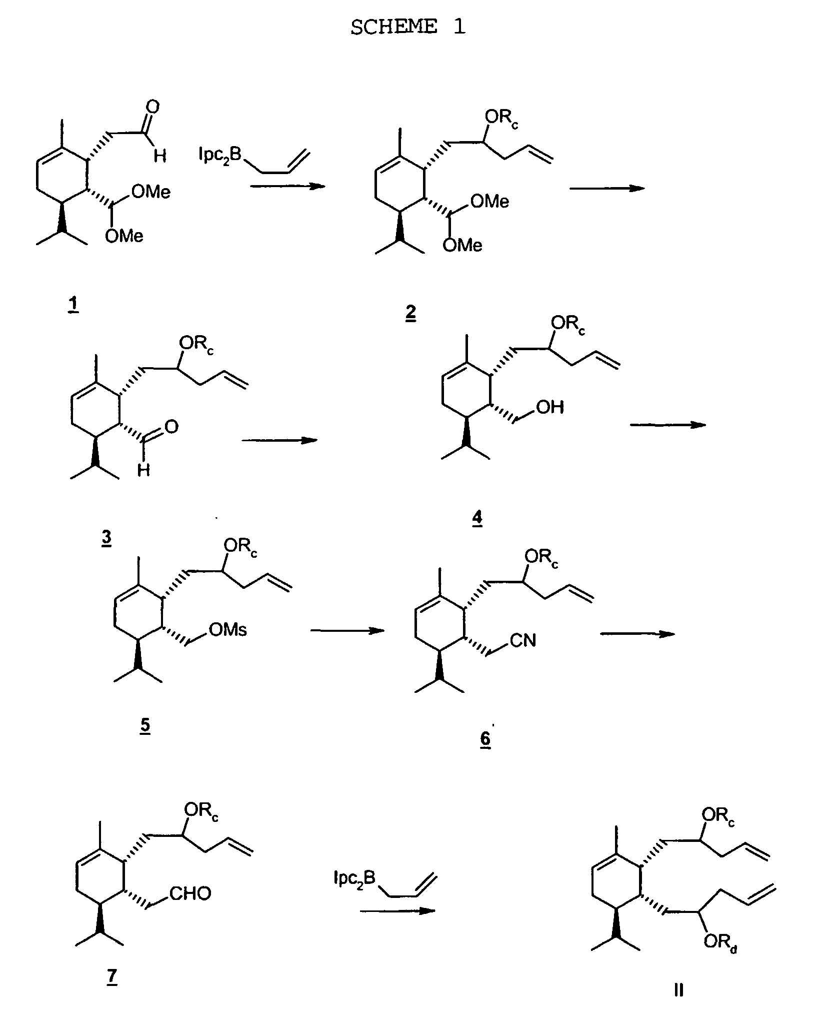

The starting compound **1** was submitted to Brown's stereoselective allylation reaction, (J. Org. Chem. ***1982**, 47, 5065)* generating the oxygenated stereocenter of the compound **2**. The allylation reaction proceeds with good to excellent stereocontrol in favor of the desired stereoisomer, just by choosing the suitable absolute stereochemistry of the alpha-pinene-derived reagent [(1-Ipc from (-)-alpha-pinene or d-Ipc from (+)-alpha-pinene]. After standard alcohol protection, such as t-butyldiphenylsilylation, an efficient and well established sequence of steps - dimethylacetal hydrolysis; aldehyde reduction; alcohol mesylation; mesylate substitution with cyanide; nitrile reduction to aldehyde- led to the homologated aldehyde 7, on which the same allylation procedure described above was applied. The allylation reaction proceeds on aldehyde 7 with good to excellent stereocontrol in favor of the desired stereoisomer, just by choosing the suitable absolute stereochemistry of the alpha-pinene-derived reagent [(1-Ipc from (-)-alpha-pinene or d-Ipc from (+)-alpha-pinene]. The starting compounds of formula 1 are known or can be obtained from known compounds in a manner analogous to that described for example in Tetrahedron Lett. **1999**, 40, 153.

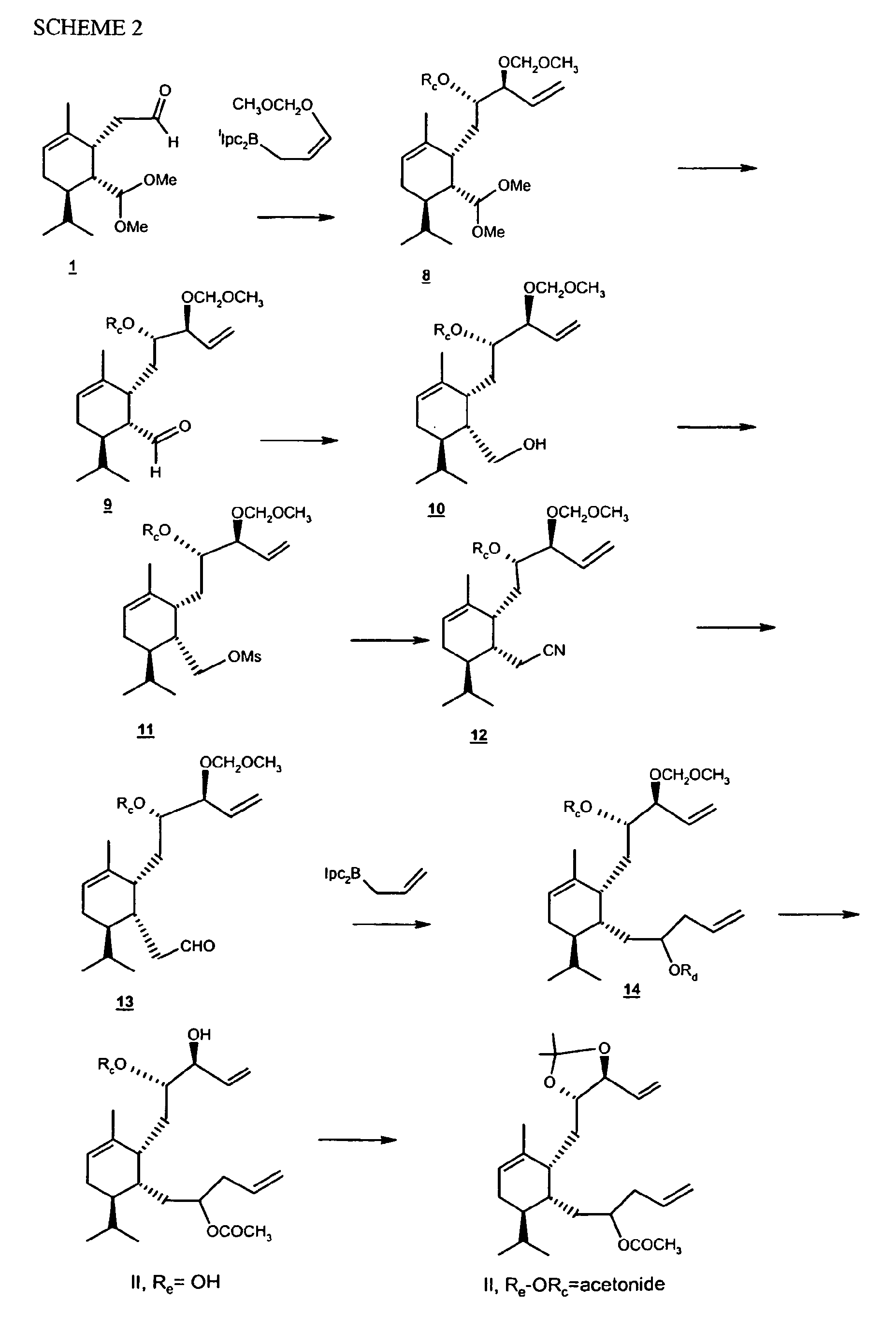

Addition of the oxyallyl borane [(1-Ipc from (-)-alpha-pinene] to aldehyde 1 gave the alcohol 8 (Rc=H) in good to excellent stereocontrol. Choosing the opposite absolute stereochemistry of the alpha-pinene-derived reagent [d-Ipc from (+)-alpha-pinene] gives access to the opposite diastereoisomer (OR_{c} up, OCH₂OCH₃ down).

Protection of the secondary alcohol as a tert-butyldiphenylsilylether, followed by the reaction sequence already mentioned above for the transformation **of 2** to **7** (deprotection of the acetal, reduction of the liberated aldehyde and C₁-chain elongation via substitution of the mesylate by cyanide) gave after reduction the aldehyde **13.** Addition of the desired allyl borane gave the secondary alcohol **(14,** R_{d}= OH), which was protected with acetic anhydride, yielding the acetate **(14,** R_{d}= COCH₃). Deprotection of the MOM-group (CH₃OCH₂-) leads to free allylic alcohol II (Rₑ=OH). As stated above, also in this last allylation reaction proceeds with good to excellent stereocontrol in favor of the desired stereoisomer, just by choosing the suitable absolute stereochemistry of the alpha-pinene-derived reagent [(1-Ipc from (-)-alpha-pinene or d-Ipc from (+)-alpha-pinene].

In alternative, an acetonide group is introduced to constrain one side chain. Deprotection of the intermediate and subsequent protection of the resultant diol (II, R_{c}=H, Rₑ=OH) gives the acetonide of formula II, wherein Rₑ and the OR_{c} group taken together form an acetonide ring.

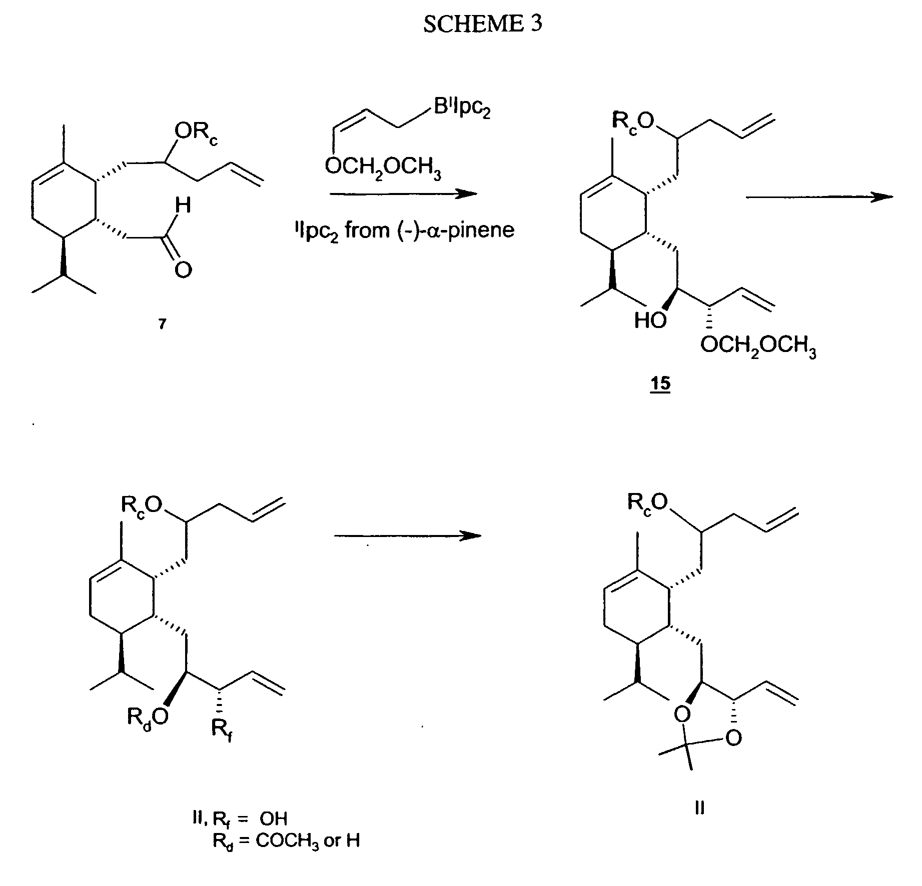

The compounds formula II wherein R_{f} is hydroxy group are prepared starting from the aldehyde 7 with the desired configuration through reactions analogous to those mentioned above. The compounds of formula II can be converted into the corresponding diol derivative by deprotection (II, R_{d}=H, R_{f}=OH), which can be further transformed into the acetonide derivative (II, R_{f}-OR_{d}= acetonide).

### BIOLOGICAL TESTS

### Cytotoxicity

A2780 cells (2000/well) were seeded in multiwell plates (96 wells) in the presence of 200 µl of the complete medium RPMI 1640 + 10% FCS. After 24 h, the cells were treated with the compounds: the compounds' solution (200 x) was prepared in DMSO 100% and 1 µl/well was added. 5 scalar concentrations for each compound were tested in four replicates. The cells were incubated at 37°C, 5% CO₂ for 72 h.

Colorimetric assay (SRB: sulforhodamine B): cell cultures were fixed with trichloroacetic acid, stained with 0.4% SRB dissolved in 1% acetic acid. Unbound dye was removed by four washes with 1% acetic acid and protein-bound dye was extracted with 10mM Tris base for determination of optical density in a 96-well microtiter plate reader. IC₅₀ and IC₉₀ (concentration inhibiting cell proliferation by 50 or 90 %) were determined by data analysis in the Microsoft Excel 97 program.

### Effect on cell cycle progression

Human colon carcinoma HCT116 cells were seeded in culture flasks and treated 24 h after incubation at 37°C. At the end of the treatment (24 or 48 or 72 hours), cells were counted and resuspended in propidium iodide (PI) staining solution (0.1 % sodium citrate, 0.1 % nonidet P40, 6.5 µg/ml Rnasi A, 50 µg/ml PI). After incubation in the dark at room temperature for at least 30 minutes, samples were then analyzed for cell cycle on FacScan (Becton Dickinson) flow cytometer.

Human colon carcinoma HT29 cells were also used to study the cytotoxicity of the compounds of the present invention.

**Table 1 Cytotoxicity tests: IC₅₀ values on several different tumor cell lines (A2780; HCT116; HT29)**

| Compound prepared in Example | IC₅₀ [µM] (A2780) | IC₅₀ [µM] (HCT116) | IC₅₀ [µM] (HT29) |
|---|---|---|---|
| 12 | 4 | 12 | - |
| 16 | 40 | 35-60 | 30 |
| 19 | 4 | 5 | 6 |
| 27 | <5 | 10-25 | 7 |
| 34 | 4 | 4 | 5 |

### Microtubule assembly and disassembly assay.

Pig brain tubulin was prepared by two cycles of assembly and disassembly and it was stored in liquid nitrogen in Microtubule Assembly Buffer (MAB: 0.1 M MES, 2.5 mM EGTA, 0.5 mM MgSO₄, 0.1 mM EDTA, 0.1 mM DTT pH 6.4). Assembly was monitored by the method of Gaskin et al.(Gaskin F, Cantor CR, Shelanski M L, 1974,: Turbidimetric studies of the in vitro assembly and disassembly of porcine neurotubules. J. Mol. Biol. 89: 737-758). The cuvette (1 cm path) containing 0.5 mg/ml tubulin and 1 mM GTP was shifted to 37 °C and continuous turbidity measurements were made at 340 nm on a spectrophotometer equipped with an automatic recorder and a thermostatically regulated sample chamber. After 30 min CaCl₂ (5 mM) was added and disassembly was monitored for 10 min as decreased turbidity. Scalar doses of test compounds were monitored at regular intervals of 15 min.

Data were expressed as percentage of reassembly induced by the tested compounds and the dose effecting tubulin assembly by 50% or 90% at 37 °C (ED₅₀ and ED₉₀) was calculated on this curve.

**Table 2. Tubulin polymerizing activities: ED₅₀ = effective dose that induces 50% tubulin polymerisation; ED₉₀= effective dose that induces 90% tubulin polymerisation**

| Compound prepared in Example | ED₅₀ [µM] | ED₉₀ [µM] |
|---|---|---|
| **12** | 2.0 | 10.0 |
| **16** | 0.2 | 1.2 |
| **19** | 5.0 | 16.0 |
| **27** | 3.0 | 7.0 |
| **30** | 1.0 | 1.7 |
| **34** | 1.0 | 1.8 |

Compounds of formula I of the invention show enhanced antitumor activity and acceptable toxicity. They are useful as antitumour agents in the prevention, treatment and/or control of cancer, for instance in the treatment of leukemia and solid tumors, such as colon, colo-rectal, ovarian, mammary, prostate, lung, kidney and also melanoma tumors. A human can be treated by a method comprising administering thereto a therapeutically effective amount of a compound of the invention. The invention therefore provides a method of treating a patient in need of an antitumour agent, which method comprises the administration thereto of a compound as defined above. The condition of the human patient can thus be improved. The invention also provides the use of a compound of the invention as defined above in the manufacture of a medicament for use as an antitumour agent.

The dosage range adopted will depend on the route of administration and on the age, weight and condition of the patient being treated. The compound of formula (I) is typically administered by parenteral route, for example intramuscularly, intravenously or by bolus infusion. A suitable dose range is from 1 to 1000 mg of equivalent per m² body surface area of active drug, for instance from 10 to 500 mg/m².

The compounds of formula (I) may be formulated into a pharmaceutical composition together with a pharmaceutically carrier or diluent. The invention therefore further provides a pharmaceutical composition which comprises a pharmaceutically acceptable diluent or carrier and, as an active ingredient, a compound as defined above. The pharmaceutical compositions of the invention are prepared by conventional methods and are adminstered in a pharmaceutically acceptable form. For example, the solid oral forms may contain, together with the active compound, diluents, e.g. lactose, dextrose, saccharose, sucrose, cellulose, corn starch or potato starch; lubricants, e.g. silica, talc, stearic, magnesium or calcium stearate, and/or polyethylene glycols; binding agents, e.g. starches, arabic gum, gelatine, methylcellulose, carboxymethylcellulose or polyvinyl pyrrolidone; disaggregating agents, e.g. a starch, alginic, alginates or sodium starch glycolate; effervescing mixtures; dyestuffs; sweeteners; wetting agents such as lecithin, polysorbates, laurylsulphates; and, in general, non-toxic and pharmacologically inactive substances used in pharmaceutical formulations. Said pharmaceutical preparations may be manufactured in known manner, for example, by means of mixing, granulating, tabletting, sugar-coating, or film-coating processes.

The liquid dispersions for oral administration may be e.g. syrups, emulsions and suspensions.

The syrups may contain as carrier, for example, saccharose or saccharose with glycerine and/or mannitol and/or sorbitol.

The suspensions and the emulsions may contain as carrier, for example, a natural gum, agar, sodium alginate, pectin, methylcellulose, carboxymethylcellulose, or polyvinyl alcohol.

The suspension or solutions for intramuscular injections may contain, together with the active compound, a pharmaceutically acceptable carrier, e.g. sterile water, olive oil, ethyl oleate, glycols, e.g. propylene glycol, and, if desired, a suitable amount of lidocaine hydrochloride. The solutions for intravenous injections or infusions may contain as carrier, for example, sterile water or preferably they may be in the form of sterile, aqueous, isotonic saline solutions or they may contain as a carrier propylene glycol.

The suppositories may contain together with the active compound a pharmaceutically acceptable carrier, e.g. cocoa butter, polyethylene glycol, a polyoxyethylene sorbitan fatty ester surfactant or lecithin.

Typically the pharmaceutical compositions are formulated for parenteral administration, for example by dissolution in water for injection or physiological saline.

### Example 1

### (R)-1-((1R,5R,6R)-6-Dimethoxyethyl-5-isopropyl-2-methyl-cyclohex-2-enyl)-pent-4-en-2-ol.

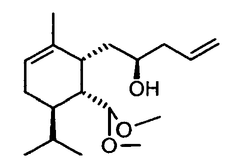

Allyl magnesium bromide (1M in Et₂O, 610 µl, 0.61 mmol, 1.2 eq) was added slowly to a stirred 0.86 M solution of ¹Ipc₂BOMe (¹Ipc derived from (-)-alpha-pinene) in THF (830 µl, 0.71 mmol, 1.4 eq) at 0 °C in an oven dried Schlenck tube under an argon atmosphere. The mixture was stirred at ambient temperature for 1 h, then cooled to -78 °C and a solution of 149 mg (0.51 mmol) of [(1R,5R,6R)-6-dimethoxymethyl-5-isopropyl-2-methyl-cyclohex-2-enyl]-acetaldehyde, prepared as described in Ceccarelli S. et al, Tetrahedron Lett. 1999, 40, 153, dissolved in 2 ml of anhydrous ether, was added dropwise via syringe. The reaction mixture was stirred at -78 °C for 6 h, then warmed to room temperature and quenched with 250 µl of 6M NaOH and 200 µl of 35 % H₂O₂. The mixture became hot spontaneously and was left standing over night. The title product was isolated and purified by intensive flash chromatography (Hex / EtOAc 4 + 1), R_{f} = 0.43. Yield: 117 mg (0.39 mmol, 77 %) as colourless oil.
*R*_{f} = 0.43 (hexane/EtOAc 4:1); ¹H NMR (200 MHz, CDCl₃): δ = 5.98-5.77 (m, 1H), 5.36 (m, 1H), 5.16-5.12 (m, 1H), 5.07 (br s, 1H), 4.36 (d, *J* = 5.4 Hz, 1H), 3.95-3.86 (m, 1H), 3.38 (s, 6H), 2.46-1.19 (m, 14H), 0.94 (d, *J* = 6.6 Hz, 3H), 0.84 (d, *J* = 6.6 Hz, 3H); ¹³C NMR (50.3 MHz, CDCl₃): δ = 136.7, 135.6, 121.1, 116.8, 106.9, 68.4, 54.7, 54.4, 42.9, 40.2, 37.1, 36.0, 34.7, 27.0, 24.4, 22.2, 21.0, 17.1; IR (CCl₄): *v*= 3590, 3480, 3064, 2945, 2919, 2820, 1516, 1457, 1438, 1380, 1361, 1155, 1105, 1065, 910; [α]_{D}²⁰ = +63.6 (c = 1.16, EtOAc); HRMS (ESI): *m*/*z*: calcd for C₁₈H₃₂NaO₃: 319.2249 [M⁺Na]⁺; found: 319.2241.

### Example 2

### tert-Butyl-[(R)-1-((1R,5R,6R)-6-dimethoxmethyl-5-isopropyl-2-methyl-cyclohex-2-enylmethyl)-but-3-enyloxy]-diphenyl-silane

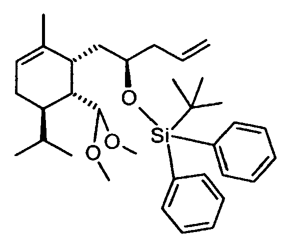

184 mg (0.62 mmol) of the compound prepared in example 1 were dissolved in 5 ml of dry CH₂Cl₂ and imidazole (211 mg, 3.11 mmol, 5 eq) is added. The solution was cooled to 0 °C and 318 µl (1.24 mmol, 2.0 eq) of TBDPSCI (tert-butyl-diphenyl-silylchloride) were slowly added. The reaction mixture was stirred at 0 °C for 90 min, then left stirring at room temperature over night, until complete reaction (TLC control). Then, the solvent was evaporated and the residue submitted to flash chromatography (Hex / EtOAc 25+1), R_{f} = 0.4. Yield: 332 mg (0.62 mmol, quant.) of the title compound as colourless oil.
R_{f} = 0.40 (hexane/EtOAc 25:1); ¹H NMR (200 MHz, CDCl₃): δ = 7.78-7.76 (m, 4H), 7.72-7.49 (m, 6H), 5.76-5.59 (m, 1H), 5.24 (m, 1H), 4.90-4.73 (m, 2H), 4.09 (d, *J* = 5.6 Hz, 1H), 4.06-3.98 (m, 1H), 3.19 (s, 3H), 3.17 (s, 3H), 2.47-2.41 (m, 1H), 2.14-2.07 (m, 2H), 1.93-1.72 (m, 6H), 1.65 and 1.64 (s, 3H), 1.42-1.22 (m, 1H), 1.06 (s, 9H), 0.84 (d, *J* = 6.8 Hz, 3H), 0.75 (d, *J*= 6.6 Hz, 3H); ¹³C NMR (50.3 MHz, CDCl₃): δ = 138.6, 136.1 (2C), 136.0 (2C), 135.3, 135.1, 134.8, 129.2 (2C), 127.3 (2C), 127.2 (2C), 120.5, 116.1, 107.6, 72.5, 54.7, 54.6, 42.3, 41.3, 38.1, 35.5, 35.1, 27.1, 27.1 (3C), 24.1, 22.9, 21.5, 19.4, 16.3; IR (CCl₄): ν = 3060, 3040, 2943, 2917, 2840, 1465, 1420, 1381 1320, 1105, 1075, 905; [α]_{D}²⁰ = +46.4 (c = 1.04, EtOAc); HRMS (ESI): *m*/*z:* calcd for C₃₄H₅₀NaO₃Si: 557.3427 [*M*+Na]⁺; found: 557.3413.

### Example 3

### (1R,2R,6R)-2-[(R)-2-(tert-Butyl-diphenyl-silyloxy)-pent-4-enyl]-6-isopropyl-3-methyl-cyclohex-3-enecarbaldehyde.

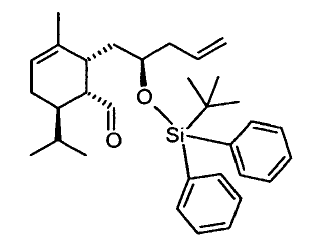

264 mg (0.49 mmol) of the compound prepared in example 2 were dissolved in 5 ml of a mixture of AcOH / H₂O / THF 3+1+1. The mixture was stirred at ambient temperature for 15 h until TLC analysis showed complete conversion (Hex / EtOAc 9+1, R_{f}= 0.68). Ether, 5 g of NaHCO₃ and 10 ml of water were added and vigorous stirring was continued until no more gas evolves. The product was extracted with 3 portions of ether, the combined organic layers dried (Na₂SO₄), the solvent was distilled and pure title compound was obtained. Yield: 242 mg (0.49 mmol, quant.), colourless oil.
¹H NMR (200 MHz, CDCl₃): δ = 9.55 (d, *J =* 3.6 Hz, 1H), 7.71-7.58 (m, 4H), 7.48-7.29 (m, 6H), 5.72-5.51 (m, 1H), 5.30 (m, 1H), 4.99-4.79 (m, 2H), 3.79-3.69 (m, 1H), 2.43-1.01 (m, 22H), 0.88 (d, *J =* 6.7 Hz, 3H), 0.74 (d, *J* = 6.6 Hz, 3H); ¹³C NMR (50.3 MHz, CDCl₃): δ= 206.5, 136.1, 135.9 (4C), 134.2, 134.1, 134.0, 129.7, 129.6, 127.6 (2C), 127.5 (2C), 121.6, 117.5, 72.1, 53.1, 41.6, 36.9, 35.7, 35.3, 27.1 (3C), 26.3, 23.8, 22.3, 20.6, 19.3, 16.9; IR (CCl₄): *ν* = 3060, 2945, 2918, 2842, 2700, 1713, 1465, 1458, 1420, 1382, 1363, 1105, 1061, 910; [α]_{D}²⁰ = +28.4 (c = 1.22, EtOAc); HRMS (ESI): *m*/*z:* calcd for C₃₂H₄₄NaO₂Si: 511.3008 [*M*+Na]⁺; found: 511.2996.

### Example 4

### {(1R,2R,6R)-2[(R)-2-(tert-Butyl-diphenyl-silyloxy)-pent-4-enyl]-6-isopropyl-3-methyl-cyclohex-3-enyl}-methanol

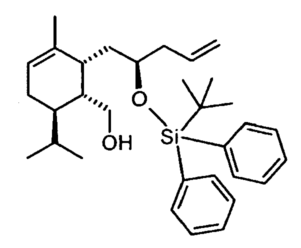

242 mg (0.49 mmol) of the compound prepared in example 3 were dissolved in 2 ml of EtOH. 28 mg (0.75 mmol, 1.5 eq) of NaBH₄ were added and the reaction mixture stirred at ambient temperature for 45 min. Then, another 10 mg (0.26 mmol, 0.5 eq) of NaBH₄ were added and stirring continued for 70 min. Then, 270 mg (5 mmol) of NH₄Cl were added, after 5 min, this mixture was diluted with ether. The ethereous phase was dried (Na₂SO₄), filtered over Celite, and the filtrate evaporated. The residue was purified by flash chromatography (Hex / EtOAc 4+1, R_{f}= 0.31). Yield: 176 mg (0.358 mmol, 73 %) of the title compound as colourless oil.
*R*_{*f*} = 0.21 (hexane/EtOAc 25:1); ¹H NMR (200 MHz, CDCl₃): δ = 7.77-7.57 (m, 4H), 7.45-7.34 (m, 6H), 5.92-5.75 (m, 1H), 5.26 (br s, 1H), 5.05-4.92 (m, 2H), 4.08-3.95 (m, 1H), 3.65 (dd, *J =* 10.9, 5.6 Hz, 1H), 3 .48-3.3 (m, 1H), 2.30 (t, *J =* 5.8 Hz, 2H), 1.90-0.91 (m, 21H), 0.85 (d, *J* = 6.8 Hz, 3H), 0.79 (d, *J* = 6.8 Hz, 3H); ¹³C NMR (75.5 MHz, CDCl₃): δ = 137.4, 136.0 (4C), 135.2, 134.7, 134.4, 129.6 (2C), 127.5 (4C), 121.5, 117.2, 72.3, 62.4, 41.7, 41.3, 36.9, 36.0, 34.7, 27.1 (4C), 24.1, 23.4, 21.2, 19.4, 16.2; IR (CCl₄): *ν* = 3615, 3060, 2945, 2918, 2880, 2842, 1422, 1381, 1363, 1105, 1058; [α]D²⁰ = +67.5 (c = 0.99, EtOAc); HRMS (ESI): *m*/*z:* calcd for C₃₂H₄₇O₂Si: 491.3345 [*M*+H]⁺; found: 491.3341.

### Example 5

### Methanesulfonic acid (1R,2R,6R)-2-[(R)-2-(tert-butyl-diphenyl-silyloxy)-pent-4-enyl]-6-isopropyl-3-methyl-cyclohex-3-enylmethyl ester

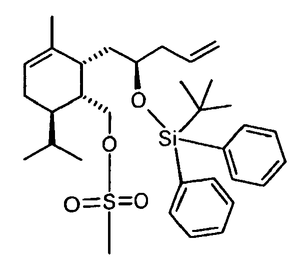

162 mg (0.33 mmol) of the compound prepared in example 4 were dissolved in 3 ml of dry CH₂Cl₂. The mixture was cooled to - 40 °C, 91 µl (67 mg, 0.66 mmol, 3 eq) of Et₃N and 40 µl (0.50 mmol, 1.5 eq) of MsCl (mesyl chloride) were added via syringe and the reaction was warmed to room temperature over 75 min. Then, NaHCO₃ sat. was added, the acqueous phase was extracted with 3 portions of CH₂Cl₂, the combined organic layers were dried over Na₂SO₄, the solvent was distilled and the product was purified by flash chromatography (Hex / EtOAc 4+1, R_{f} = 0.63). Yield: 188 mg of the title compound (0.33 mmol, quant.) as colourless oil.
R_{f}= 0.63 (hexane/EtOAc 4:1); ¹H NMR (200 MHz, CDCl₃): δ= 7.65-7.59 (m, 4H), 7.44-7.30 (m, 6H), 5.88-5.67 (m, 1H), 5.23 (br s, 1H), 5.02-4.88 (m, 2H), 4.21 (dd, *J*= 10.0, 6.5 Hz, 1H), 3.98 (dd, *J* = 10.0, 8.5 Hz, 2H), 2.86 (s, 3H), 2.31-2.26 (m, 3H), 2.04-0.99 (m, 19H), 0.82 (d, *J* = 6.0 Hz, 3H), 0.80 (d, *J* = 6.0 Hz, 3H); ¹³C NMR (75.5 MHz, CDCl₃): δ = 136.3, 135.9 (4C), 134.7, 134.4 (2C), 129.6 (2C), 127.6 (4C), 121.5, 117.5, 72.1, 70.1, 41.3, 39.0, 37.3, 37.0, 36.3, 34.8, 27.3, 27.1 (3C), 23.7, 23.2, 21.0, 19.4, 17.0; IR (CCl₄): *ν* = 3070, 2960, 2930, 2858, 1471, 1428, 1389, 1368, 1348, 1329, 1180, 1110, 1062; [α]_{D}²⁰ = +58.1 (c = 0.92, EtOAc); HRMS (ESI): *m*/*z*: calcd for C₃₃H₅₂NO₄SiS: 586.3386 [*M*+NH₄]⁺; found: 586.3368.

### Example 6

### {(1R,2R,6R)-2-[(R)-2-(tert-Butyl-diphenyl-silyloxy)-pent-4-enyl]-6-isopropyl-3-methyl-cyclohex-3-enyl}-acetonitrile.

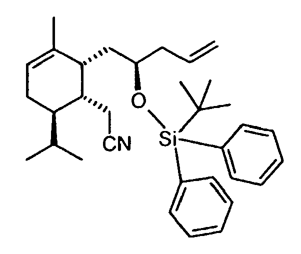

162 mg (285 µmol) of the compound prepared in example 5 were dissolved in 3 ml of dry acetonitrile. 56 mg (855 µmol, 3 eq) of KCN and 226 mg (855 µmol, 3 eq) of 18-crown-6 were added. The reaction mixture was heated to 80 °C for 6 h. The solvent was evaporated under reduced pressure, the residue was dissolved in Hex / EtOAc 9+1 and purified by flashchromatography (Hex / EtOAc 9+1, R_{f} = 0.60), yield: 136 mg (272 µmol, 95 %) of the title compound as colourless oil.
*R*_{f} = 0.40 (hexane/EtOAc 14:1); ¹H NMR (200 MHz, CDCl₃): δ = 7.71-7.69 (m, 4H), 7.47-7.33 (m, 6H), 5.84-5.70 (m, 1H), 5.22 (br s, 1H), 5.05-4.92 (m, 2H), 3.95-3.86 (m, 1H), 2.27-1.27 (m, 15H), 1.05 (s, 9H), 0.83 (d, *J* = 6.8 Hz, 3H), 0.79 (d, *J* = 6.8 Hz, 3H); ¹³C NMR (50.3 MHz, CDCl₃): δ = 135.8 (4C), 135.4, 134.3, 134.2, 134.1, 129.5 (2C), 127.4 (4C), 121.2, 119.4, 117.6, 71.8, 40.9, 38.5, 36.3, 36.1, 35.9, 27.2, 27.0 (3C), 23.4, 22.8, 20.8, 19.2, 17.8, 17.5; IR (CCl₄): *ν* = 3070, 2955, 2922, 2890, 2856, 1715, 1470, 1460, 1425, 1388, 1369, 1110, 1070, 915; [α]_{D}²⁰ = +66.4 (c = 1.28, EtOAc); HRMS (ESI): *m*/*z*: calcd for C₃₃H₄₅NaNOSi: 522.3168 [*M*+Na]⁺; found: 522.3179.

### Example 7

### {(1R,2R,6R)-2-[(R)-2-(tert-Butyl-diphenyl-silyloxy)-pent-4-enyl]-6-isopropyl-3-methyl-cyclohex-3-enyl}-acetaldehyde.

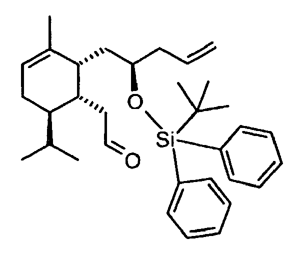

136 mg of the compound prepared in example 6 were dissolved in 3 ml of toluene / hexane 1+2. At -78 °C, 2.7 ml (2.7 mmol, 10 eq) of DIBA1H (Di-isobutyl aluminium hydride, 1.0 M in hexane) were added. After stirring 45 min at -78 °C the reaction was quenched with 1.5 ml of EtOAc and 5 ml of 1 M tartaric acid. Extraction with 3 portions of CH₂Cl₂, washing the combined organic phases with IN HCl, drying (Na₂SO₄) and purification by flash chromatography (Hex / EtOAc 9+1, R_{f} = 0.60), 107 mg (121 mmol, 78 %) of the title compound were obtained as colourless oil.
*R*_{f}= 0.39 (hexane/EtOAc 14:1); ¹H NMR (200 MHz, CDCl₃): δ= 9.66 (s, 1H), 7.78-7.71 (m, 4H), 7.457.35 (m, 6H), 5.91-5.71 (m, 1H), 5.26 (br s, 1H), 5.07-4.90 (m, 2H), 3.91 (q, *J =* 5.9 Hz, 1H), 2.38-2.03 (m, 5H), 1.99-0.93 (m, 19H), 0.87 (d, *J =* 6.7 Hz, 3H), 0.75 (d, *J=* 6.7 Hz, 3H); ¹³C NMR (75.5 MHz, CDCl₃): *δ*= 203.1, 136.6, 135.9 (4C), 134.6, 134.5, 134.3, 129.6 (2C), 127.5 (4C), 121.3, 117.4, 72.1, 44.2, 41.2, 38.9, 37.0, 36.4, 34.3, 27.4, 27.1 (3C), 23.7, 23.1, 21.2, 19.4, 17.2; IR (CCl₄): *ν*= 3060, 2943, 2917, 2880, 2842, 2695, 1720, 1465, 1455, 1420, 1382, 1365, 1103, 1095, 1060, 910; [α]_{D}²⁰ = +53.3 (c = 1.14, EtOAc).

### Example 8

### (R)-1-{(1R,2R,6R)-2-[(R)-2-(tert-Butyl-diphenyl-silyloxy)-pent-4-enyl]-6-isopropyl-3-methyl-cyclohex-3-enyl}-pent-4-en-2-ol.

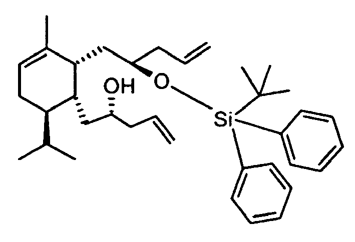

Allyl magnesium bromide (1.0 M in Et₂O, 150 µl, 0.15 mmol, 3 eq) was added to a solution of 170 µl (0.17 mmol, 3.3 eq) of ¹Ipc₂BOMe (¹Ipc derived from (-)-alpha-pinene, 1.0 M in THF) at 0 °C. The mixture was stirred at room temperatur for 1 h. After 1 h, it was cooled to - 78 °C and a solution of 25 mg (50 µmol) of the compound prepared in example 7 in 200 µl of dry ether were added. The mixture was stirred at -78 °C for 3 h 45 min, then it was warmed to room temperature and quenched with 200 µl of 30 % H₂O₂ and 250 µl of 6M NaOH. Stirring was continued at ambient temperature for 40 min, water and ether were added and the product was isolated by extraction with 3 portions of ether, drying (Na₂SO₄) and intensive flash chromatography (Hex / EtOAc 4+1, R_{f} = 0.59). Yield: 15 mg (28 µmol, 55 %) of the title compound as a colourless oil.
*R*_{f} *=* 0.59 (hexane/EtOAc 4:1); ¹H NMR (200 MHz, CDCl₃): δ= 7.73-7.56 (m, 4H), 7.46-7.27 (m, 6H), 5.91-5.70 (m, 2H), 5.49-4.92 (m, 5H), 4.17-3.89 (m, 1H), 3.67-3.62 (m, 1H), 2.51-1.92 (m, 5H), 1.82-1.19 (m, 13H), 1.09 (s, 9H), 0.82 (d, *J* = 6.7 Hz, 3H), 0.76 (d, *J* = 6.7 Hz, 3H); ¹³C NMR (50.3 MHz, CDCl₃): *δ*□ = 136.9, 135.9 (4C), 135.0, 134.9, 134.6, 134.5, 129.5 (2C), 127.4 (4C), 121.1, 118.0, 117.0, 72.2, 68.3, 41.9, 41.5, 38.5, 37.1, 35.8, 35.4, 35.2, 27.1, 27.0 (3C), 23.9, 23.1, 21.2, 19.3, 17.5; IR (CCl₄): *ν*= 3595, 3078, 2960, 2934, 2899, 2860, 1642, 1475, 1465, 1430, 1389, 1371, 1110, 1069, 917; [α]_{D}²⁰ = +38.8 (c = 0.84, EtOAc); HRMS (ESI): *m*/*z*: calcd for C₃₆H₅₂NaO₂Si: 567.3634 [M+Na]⁺; found: 567.3665.

### Example 9

### Acetic acid (R)-1-{(1R,2R,6R)-2-[(R)-2-(tert-butyl-diphenyl-silyloxy)-pent-4-enyl]-6-isopropyl-3-methyl-cyclohex-3-enylmethyl}-but-3-enyl ester.

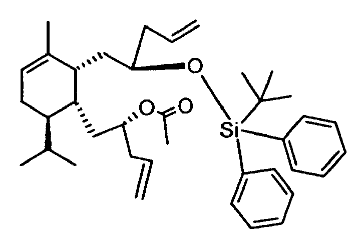

78 mg (0.14 mmol) of the compound prepared in example 8 were treated with acetic anhydride (41 µl, 0.44 mmol, 3 eq) and 4-DMAP (4-dimethylaminopyridine, 2 mg) in 500 µl of pyridine at 0 °C. Stirring was continued until the reaction was complete by TLC (3 h). The mixture was diluted with EtOAc, washed with saturated KHSO₄ aqueous solution, dried (Na₂SO₄) and evaporated. The residue was purified by flash chromatography (Hexane / EtOAc 14+1, R_{f}= 0.65). Yield: 75 mg (0.13 mmol, 93 %) of the title compound as colourless oil.
*R*_{f} = 0.58 (hexane/EtOAc 14:1); ¹H NMR (200 MHz, CDCl₃): δ = 7.99-7.65 (m, 4H), 7.45-7.28 (m, 6H), 5.91-5.62 (m, 2H), 5.19-4.87 (m, 6H), 3.91 (q, *J =* 5.7 Hz, 1H), 2.30-2.01 (m, 5H), 1.97 (s, 3H), 1.85-1.14 (m, 11H), 1.06 (s, 9H), 0.95-0.84 (m, 1H), 0.76 (d, *J* = 6.6 Hz, 3H), 0.72 (d, *J =* 6.6 Hz, 3H); ¹³C NMR (50.3 MHz, CDCl₃): *δ* = 170.4, 135.8 (4C), 135.0, 134.5, 134.4 (2C), 133.6, 129.4 (2C), 127.3 (4C), 121.1, 117.6, 117.0, 71.9, 71.2, 41.4, 39.2, 39.0, 36.6, 34.8, 33.5, 31.5, 27.2, 27.0 (3C), 23.6, 22.9, 21.1, 20.9, 19.3, 19.2; IR (CCl₄): *ν*= 3050, 2934, 2911, 2831, 1726, 1460, 1450, 1415, 1375, 1359, 1096, 1090, 1052, 900; [α]_{D}²⁰ = +27.9 (c = 1.26, EtOAc); HRMS (ESI): *m*/*z*: calcd for C₃₈H₅₄NaO₃Si: 609.3740 [*M*+Na]⁺; found: 609.3746.

### Example 10

### 11-Acetoxy-1-isopropyl-4-methyl-6-(tert-butyl-diphenyl-silyloxy)-1, 2, 4a, 5, 6, 7, 10, 11, 12, 12a-decahydro-benzocyclodecene.

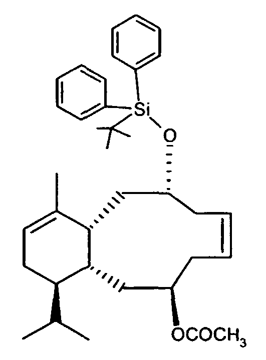

6 mg (10 µmol) of the compound prepared in example 9 was dissolved in 1 ml of dry degassed dichloromethane. 2 mg (2 µmol, 0.2 eq) of RCM catalyst A were dissolved in 500 µl of dry degassed dichloromethane and this solution was slowly added to the solution of the compound prepared in example 9 over 2 h under an argon atmosphere. The mixture was then stirred over night under an argon atmosphere. Another 1 mg (1 µmol, 0.1 eq) was dissolved in 250 µl of DCM and slowly added via syringe pump over 30 min. Stirring at ambient temperature was continued until no more starting material can be detected by TLC. Then, the solvent was distilled and the residue was submitted to flash chromatography (Hexane/EtOAc 9+1, Rf = 0.53). Yield of the title compound: 5 mg (9 µmol, 90 %).
*R*_{f} = 0.43 (hexane/EtOAc 14:1); ¹H NMR (400 MHz, CDCl₃): δ = 7.72-7.62 (m, 4H), 7.44-7.33 (m, 6H), 5.86 (dt, *J* = 11.1, 4.7 Hz, 1H), 5.51 (ddd, *J* = 11.7, 3.9 Hz, 1H), 5.20 (m, 1H), 5.13 (m, 1H), 4.17 (m, 1H), 2.77-2.49 (m, 2H), 2.29-2.25 (m, 1H), 2.15 (m, 1H), 2.05-0.85 (m, 23H), 0.89-0.85 (m, 1H), 0.81 (d, J= 6.6 Hz, 3H), 0.77-0.65 (m, 1H), 0.61 (d, *J* = 6.6 Hz, 3H); ¹³C NMR (50.3 MHz, CDCl₃): *δ* = 170.4, 138.5, 135.7 (4C), 134.4 (2C), 129.5 (2C), 127.5 (4C), 126.9 (2C), 120.4, 72.8 (2C), 37.8, 37.0, 36.6, 34.7, 31.9, 31.6, 26.9 (4C), 26.2, 24.2, 23.9, 21.2, 20.9, 19.2, 14.9; IR (CCl₄): *ν* = 3070, 2958, 2926, 2856, 1740, 1460, 1427, 1368, 1110, 1067; [α]_{D}²⁰ = +41.0 (c = 1.26, EtOAc); HRMS [EI (70 eV)]: *m*/*z:* calcd for C₃₆H₅₀O₃Si: 558.3529 [*M*]⁺; found: 558.3532.

Assignment of the olefinic ³*J*_{cis} coupling constant (11.5 Hz between the protons at *δ* = 5.51 and δ = 5.86 ppm, respectively) by a 400 MHz H,H-COSY experiment.

Unequivocal assignment of *cis* stereochemistry of the double bond by detection of a NOE contact between olefinic protons in a 400 MHz NOESY experiment. 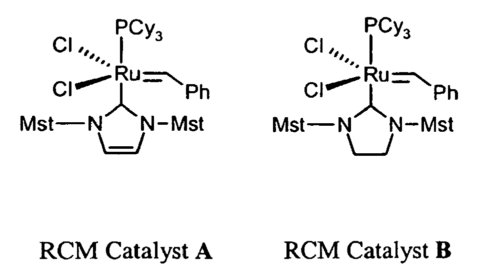 [Mst=C₆H₂-2,4,6-(CH₃)₃]

The same reaction was repeated with the RCM catalyst **B** (7% mol), in CH₂Cl₂ at room temperature, and after 168 h the title compound was isolated in 88% yield (95% after recovering starting material, stereochemistry of the double bond: 100% Z).

### Example 11

### 11-Acetoxy-6-hydroxy-1-isopropyl-4-methyl-1,2,4a,5,6,7,10,11,12,12a-decahydro-benzocyclodecene.

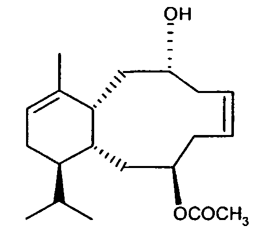

A solution of the compound prepared in example 10 (19 mg, 0.034 mmol) in THF (550 µl) was treated with I M TBAF in THF (170 µl, 5 eq.) at room temperature. The mixture was stirred for 16 h, then treated with pH 7 phosphate buffer, and extracted with EtOAc. The combined organic extracts were dried (Na₂SO₄) and evaporated to give a crude mixture, which was purified by flash-chromatography (hexane-EtOAc 4+1) to yield the title compound (10 mg, 94%).
*R*_{f}= 0.27 (hexane/EtOAc 4:1); ¹H NMR (200 MHz, CDCl₃): δ= 5.72 (dt, *J=* 11.2, 3.9 Hz, 1H), 5.53 (dt, *J*= 11.7, 4.0 Hz, 1H), 5.31-5.12 (m, 2H), 4.26-4.19 (m, 1H), 2.85-2.66 (m, 2H), 2.42-1.16 (m, 19H), 0.85 (d, *J =* 6.8 Hz, 3H), 0.67 (d, *J =* 6.8 Hz, 3H); ¹³C NMR (50.3 MHz, CDCl₃): *δ*= 170.4, 138.4, 127.9, 126.2, 121.1, 72.6, 71.3, 38.0, 37.4, 36.8, 34.7, 31.9, 31.8, 27.0, 26.4, 24.4, 24.3, 21.2, 21.0, 15.1; IR (CCl₄): *ν*= 3634, 3621, 2924, 2843, 1739, 1460, 1382, 1364; [α]_{D}²⁰ = +4.6 (c = 0.46, EtOAc); HRMS (ESI): *m*/*z:* calcd for C₂₀H₃₂NaO₃: 343.2249 [*M*+Na]⁺; found: 343.2241.

### Preparation step A: (E)-3-(1H-Imidazol-4-yl)-2-propenoic Acid Ethyl Ester.

(*E*)-3-(1*H*-Imidazol-4-yl)-2-propenoic Acid (2.0 g, 14.48 mmol) was dissolved in absolute EtOH (10 mL) under N₂. H₂SO₄ (conc., 1 ml) was added and the mixture was refluxed for 3 h until the solution was clear. The mixture was neutralized with saturated aqueous NaHCO₃ solution (pH = 7). The solution was extracted with EtOAc (3 x 20 mL) and the combined organic layers dried over Na₂SO₄. The solvent was evaporated under reduced pressure to yield the title compound (2.19 g, 13.19 mmol, 91 %) as a white solid.
¹H-NMR (CDCl₃, 200 MHz): *δ* = 1.31 (t, *J* = 7.1 Hz, 3H, CO₂CH₂CH₃), 4.22 (q, *J* = 7.1 Hz, 2H, CO₂C*H*₂CH₃), 6.44 (d, *J* = 15.7 Hz, 1H, C*H*=CH-CO₂CH₂CH₃), 7.29 (s, 1H, Ar-H), 7.60 (d, *J* = 15.7 Hz, 1H, C*H*=CH-CO₂CH₂CH₃), 7.71 (s, 1H, Ar-H), 8.30 (br s, 1H, N*H).*

### Preparation step B (E)-3-(1-Methyl-Imidazol-4-yl)-2-propenoic Acid Ethyl Ester

The compound prepared in preparation step A (2.19 g, 13.19 mmol) was dissolved in absolute CH₃CN (20 ml) under N₂. K₂CO₃ (1.0 eq., 13.19 mmol, 1.82 g) and MeI (1.0 eq., 13.19 mmol, 1.87 g) were added. The solution stirred for 24 h at room temperature in a closed flask (TLC showed starting material left). MeI (0.5 eq., 6.60 mmol, 0.91 g) were added and the solution stirred for another 48 h at room temperature in a closed flask (TLC showed minimal starting material left). CHCl₃ (20 mL) and H₂O (20 mL) were added. The layers were separated and the aqueous layer was extracted with CHCl₃ (2 x 10 mL). The combined organic layers were dried over Na₂SO₄. The solvent was evaporated under reduced pressure and the resulting residue was purified by flash chromatography (CH₂Cl₂/EtOH 97:3) to yield the title compound (714 mg, 3.96 mmol, 30%) as a white solid. R_{*f*} = 0.55 (CH₂Cl₂/EtOH 97:3); ¹H-NMR (CDCl₃, 200 MHz): *δ* = 1.28 (t, *J =* 7.1 Hz, 3H, CO₂CH₂CH₃), 3.68 (s, 3H, N-*CH*₃), 4.21 (q, *J =* 7.1 Hz, 2H, CO₂CH₂CH₃), 6.50 (d, *J =* 15.7 Hz, 1H, CH=*CH*-CO₂CH₂CH₃), 7.05 (s, 1H, Ar-H), 7.42 (s, 1H, Ar-H), 7.51 (d, *J=* 15.7 Hz, 1H, C*H*=CH-CO₂CH₂CH₃).

### Preparation step C (E)-3-(1-Methyl-Imidazol-4-yl)-2-propenoic Acid Sodium Salt

The compound prepared in preparation step B (187 mg, 1.038 mmol) was dissolved in THF/H₂O (1:1, 8 ml). NaOH (1.0 eq., 1.038 mmol, 41.5 mg) was added and the solution stirred 48 h at room temperature (TLC showed minimal starting material left and the pH was neutral, between 7-8 ). The solvent was evaporated under reduced pressure, coevaporated with benzene (5 x 5 mL) and dried at a high vacuum pump to yield the title compound as a grey-white solid, that was used without further purification.
¹H-NMR (DMSO-D₆, 200 MHz): δ = 3.60 (s, 3H, N-C*H*₃), 6.20 (d, *J* = 15.7 Hz, 1H, CH=C*H*-CO₂CH₂CH₃), 6.96 (d, *J* = 15.7 Hz, 1H, C*H*=CH-CO₂CH₂CH₃), 7.16 (s, 1H, Ar-H), 7.50 (s, 1H, Ar-H).

### Example 12

### 3-(1-Methyl-1H-imidazol-4-yl)-acrylic acid 11-acetoxy-1-isopropyl-4-methyl-1,2,4a,5,6,7,10,11,12,12a-decahydro-benzocyclodecen-6-yl ester.

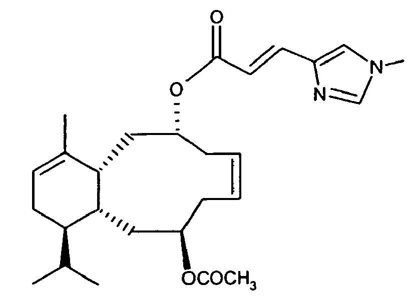

The compound prepared in the preparation step C (15.0 eq., 0.515 mmol, 90 mg) was dissolved in dry THF (5 mL) under Ar. Pivaloylchloride (15.0 eq, 0.515 mmol, 62 mg) was added and the solution stirred for 24 h at room temperature in a closed flask. The solution was filtered under Ar to separate the precipitated NaCl, the precipitate was washed with dry THF (2 x 5mL) and the solvent carefully evaporated under reduced pressure to give the corresponding crude mixed anhydride as a white suspension (checked by¹H-NMR in CDCl₃). The alcohol prepared in example 11 (1.0 eq., 0.0343 mmol, 11.0 mg) was dissolved in dry CH₂Cl₂ (2 ml) and added to the solution of the crude mixed anhydride under Ar. NEt₃ (15.0 eq., 0.515 mmol, 52 mg) and DMAP (1.0 eq., 0.0343 mmol, 4 mg) were added and the the solution stirred for 3 d at room temperature. The solvent was evaporated under reduced pressure and the resulting residue was purified by flash chromatography (Hexane/EtOAc 1:4) to yield the title compound (9.2 mg, 0.020 mmol, 59%) as an oil.
R_{*f*}= 0.25 (hexane/EtOAc 1:4); ¹H NMR (400 MHz, CDCl₃): *δ*= 7.55 (d, *J*= 15.6 Hz, 1H), 7.47 (s, 1H), 7.09 (s, 1H), 6.54 (d, *J =* 15.6 Hz, 1H), 5.74-5.66 (m, 1H), 5.62-5.55 (m, 1H), 5.45-5.39 (m, 1H), 5.34 (br s, 1H), 5.24-5.18 (m, 1H), 3.72 (s, 3H), 2.88-2.77 (m, 2H), 2.40-2.25 (m, 2H), 2.23-2.18 (m, 1H), 2.07 (s, 3H), 2.05-1.26 (m, 12H), 0.87 (d, *J=* 6.7 Hz, 3H), 0.70 (d, *J =* 6.7 Hz, 3H); ¹³C NMR (50.3 MHz, CDCl₃): *δ*= 170.4, 166.7, 137.9, 138.4, 135.9, 127.9, 126.4, 122.2, 121.0, 116.4, 73.6, 72.5, 37.5, 37.3, 34.6, 33.5, 32.7, 31.6, 29.6, 29.2, 26.9, 26.2, 24.3, 24.2, 21.2, 20.9, 15.1; IR (CCl₄): *ν* = 2960, 2850, 1745, 1705, 1640, 1450, 1440, 1380, 1345, 1295, 905; [*α*]_{D}²⁰ = -29 (c = 0.71, EtOAc); HRMS [EI (30 eV)]: *m*/*z:* calcd for C₂₇H₃₈N₂O₄: 454.2832 [*M*]⁺; found: 454.2802.

### Example 13

### (4R,4aR,6R, 11R,12aR)-11 -(tert-Butyl-diphenyl-silanoxy)-4-isopropyl-1-methyl-3,4,4a,5,6,7,10,11,12,12a-decahydro-benzocyclodecen-6-ol

The compound prepared in Example 10 (30 mg, 0.05 mmol) was dissolved in MeOH (1 mL). K₂CO₃ (17 mg, 0.10 mmol) was added and the solution stirred for 15 h at room temperature. H₂O (2mL) was added, the layers were separated and the aqueous layer was extracted with EtOAc (3x5 mL), the combined organic layers were washed with a saturated aqueous NaCl solution (2x5 mL) and the combined organic layers were dried over Na₂SO₄. The solvent was evaporated under reduced pressure and the resulting residue was purified by flash chromatography (hexane/EtOAc 9:1) to provide the title compound (26 mg, 94 %) as a colourless oil. R_{*f*} = 0.25 (hexane/EtOAc 9:1); ¹H NMR (200 MHz, CDCl₃): *δ =* 7.74-7.61 (m, 4H), 7.46-7.31 (m, 6H), 5.82 (dt, *J =* 11.3, 5.1 Hz, 1H), 5.52 (dt, *J =* 11.3, 5.1 Hz, 1H), 5.13 (br s, 1H), 4.23-4.04 (m, 2H), 2.70-2.49 (m, 2H), 2.33-2.04 (m, 3H), 1.99-1.18 (m, 13H), 1.08 (s, 9H), 0.84 (d, *J* = 6.8 Hz, 3H), 0.72 (d, *J* = 6.8 Hz, 3H); ¹³C NMR (50.3 MHz, CDCl₃): *δ* = 138.5, 135.7 (4C), 134.5, 134.4, 129.4 (2C), 127.9, 127.5 (4C), 126.6, 120.4, 72.7, 71.0, 37.9, 37.3, 36.6, 35.5, 33.7, 32.0, 29.1, 27.0 (4C), 24.3, 23.9, 21.0, 19.2, 15.3; IR (CCl₄): *ν* = 3611, 2942, 2921, 2842, 1472, 1458, 1427, 1389, 1369, 1109, 1067, 909; [α]_{D}²⁰ = +25.3 (c = 0.73, EtOAc); HRMS (ESI): *m*/*z:* calcd for C₃₄H₄₈NaO₂Si: 539.3321 [*M*+Na]⁺; found: 539.3306.

### Example 14

### (4R,4aR,11R,12aR)-11-(tert-Butyl-diphenyl-silanoxy)-4-isopropyl-1-methyl-3,4a,5,7,10,11,12,12a-octahydro-4H-benzocyclodecen-6-one:

(COCl)₂ (91 mg, 0.72 mmol) was dissolved in CH₂Cl₂ (0.5 mL) and cooled to -60 °C. DMSO (77 mg, 0.98 mmol) was added and the solution stirred for 10 min at -60 °C.

The compound prepared in Example 13 (62 mg, 0.12 mmol) in CH₂Cl₂ (0.5 mL) was added and the solution stirred for further 30 min at -60 °C. NEt₃ (199 mg, 1.97 mmol) was added, the solution was allowed to warm to 0 °C over 1 h and stirred additional 10 min at 0 °C. An aqueous phosphate buffer solution (3 mL, pH = 7) was added, the layers were separated and the aqueous layer was extracted with CH₂Cl₂ (3x5 mL) and the combined organic layers were dried over Na₂SO₄. The solvent was evaporated under reduced pressure and the resulting residue was purified by flash chromatography (hexane/EtOAc 9:1) to provide unreacted starting compound (9 mg) and the title compound (50 mg, 81 %, 95 % after recovering starting material) as colourless oils. R_{*f*}= 0.55 (hexane/EtOAc 9:1); ¹H NMR (200 MHz, CDCl₃): δ= 7.77-7.59 (m, 4H), 7.47-7.29 (m, 6H), 6.19-6.06 (m, 1H), 5.97-5.84 (m, 1H), 5.06 (br s, 1H), 3.96-3.82 (m, 1H), 3.20-2.87 (m, 3H), 2.32-1.95 (m, 4H), 1.89-1.61 (m, 5H), 1.41-1.18 (m, 5H), 1.07 (s, 9H), 0.87 (d, *J =* 6.8 Hz, 3H), 0.69 (d, *J =* 6.8 Hz, 3H); ¹³C NMR (50.3 MHz, CDCl₃): *δ* = 213.0, 139.2, 135.8 (4C), 134.0 (2C), 131.4, 129.7, 129.6, 127.6 (2C), 127.5 (2C), 123.7, 116.7, 73.0, 44.1, 38.7, 36.0, 36.7 (2C), 36.5, 32.1, 27.0 (3C), 26.6, 24.1, 22.6, 21.1, 19.2, 14.3, IR (CCl₄): *ν* = 3022, 2955, 2922, 2899, 2860, 1708, 1705, 1469, 1427, ;[*α*]_{D}²⁰ = +49.0 (c = 0.79, EtOAc); HRMS (ESI): *m*/*z:* calcd for C₃₄H₄₆NaO₂Si: 537.3165 [*M*+Na]⁺; found: 537.3139.

### Example 15

### (4R,4aR, 11R, 12aR)-11-Hydroxy-4-isopropyl-1-methyl-3,4a,5,7,10,11,12,12a-octahydro-4H-benzocyclodecen-6-one

The compound prepared in example 14 (16 mg, 0.03 mmol) was dissolved in THF (0.50 mL) and TBAF (0.06 mL, 0.06 mmol, 1.0 M in THF) was added. The reaction mixture stirred 23 h at room temperature. Additional TBAF (0.06 mL, 0.06 mmol, 1.0 M in THF) was then added and the solution stirred for further 5 h at room temperature. An aqueous phosphate buffer solution (1.0 mL, pH = 7) was added, the layers were separated and the aqueous layer was extracted with EtOAc (3x5 mL) and the combined organic layers were dried over Na₂SO₄. The solvent was evaporated under reduced pressure and the residue was purified by flash chromatography (hexane/EtOAc 8:2) to yield the title compound (10 mg, quant.) as a colourless oil. *R*_{f} = 0.17 (hexane/EtOAc 8:2); ¹H NMR (200 MHz, CDCl₃): δ = 6.01-5.84 (m, 2H), 5.23 (br s, 1H), 4.07-3.93 (m, 1H), 3.24-2.87 (m, 3H), 2.42-2.08 (m, 4H), 1.92-1.01 (m, 11H), 0.87 (d, J= 6.9 Hz, 3H), 0.71 (d, *J*= 6.9 Hz, 3H); ¹³C NMR (75.5 MHz, CDCl₃): *δ* = 212.8, 139.0, 130.3, 124.6, 119.5, 71.9, 44.2, 39.0, 38.0, 37.1, 37.0, 36.9, 32.1, 26.6, 24.2, 23.3, 21.2, 14.4; IR (CCl₄): *v*= 3627, 2960, 2951, 2904, 1709, 1460, 1442, 1389, 1371, 909; [*α*]_{D}²⁰ = -4.7 (c = 0.51, EtOAc).

### Example 16

### (E)-3-(1-Methyl-1H-imidazol-4-yl)-acrylic acid [(1R,4aR,6R,12aR)-1-isopropyl-4-methyl-11-oxo-1,2,4a,5,6,7,10,11,12,12a-decahydro-benzocyclodecen-6-enyl] ester

The compound prepared in Example 15 (9 mg, 0.03 mmol) was dissolved in CH₂Cl₂ (2 mL) and added to Mixed Anhydride (110 mg, 0.47 mmol). NEt₃ (48 mg, 0.47 mmol) and DMAP (4 mg, 0.03 mmol) were added and the solution stirred for 3 d at 40 °C. The solvent was evaporated under reduced pressure and the resulting residue was purified by flash chromatography (hexane/EtOAc 1:5) to yield the title compound (7 mg, 52 %) as a colourless oil. R_{*f*}= 0.18 (hexane/EtOAc 1:5); ¹H NMR (400 MHz, CDCl₃): δ= 7.55 (d, *J* = 15.6 Hz, 1H), 7.47 (s, 1H), 7.09 (s, 1H), 6.55 (d, *J*= 15.6 Hz, 1H), 5.97-5.88 (m, 2H), 5.26 (br s, 1H), 5.15 (d, *J*= 12.0 Hz, 1H), 3.72 (s, 3H), 3.25-3.15 (m, 1H), 3.09-2.96 (m, 2H), 2.46-2.14 (m, 4H), 1.94-1.55 (m, 7H), 1.48-1.17 (m, 3H), 0.89 (d, *J* = 6.9 Hz, 3H), 0.75 (d, *J* = 6.9 Hz, 3H);¹³C NMR (100.8 MHz, CDCl₃): *δ* = 212.7, 166.6, 139.1, 139.0. 138.6, 136.0, 130.6, 124.7, 122.3, 119.2, 116.3, 73.7, 44.1, 39.1, 37.0, 36.8 (2C), 34.1, 33.5, 29.7, 26.7, 24.2, 23.1, 21.1, 14.4; IR (CCl₄): *ν* = 2945, 2920, 2890, 1703, 1640, 1452, 1381, 1390, 1153, 1104, 905; [*α*]_{D}²⁰ = - 15.6 (c = 0.34, EtOAc); HRMS (ESI): *m*/*z*: calcd for C₂₅H₃₅N₂O₃: 411.2648 [M+H]⁺; found: 411.2648.

### Example 17

### (1R,4aR,6R,12aR)-tert-Butyl-(1-isopropyl-4-methyl-11-methylene-1,2,4a,5,6,7,10,11,12,12a-decahydro-benzocyclodecen-6-yloxy)-diphenyl-silane

Ph₃PCH₃Br (17 mg, 0.047 mmol) was dissolved in THF (0.2 mL). n-BuLi (13 µL, 0.020 mmol, 1.6 M in *n*-hexane) was added and the solution stirred for 1 h at room temperature. The compound prepared in Example 14 (8 mg, 0.016 mmol) was added in THF (600 µL) and the solution was heated to 50 °C for 12 h. H₂O (2.0 mL) was added and the layers were separated. The aqueous layer was extracted with EtOAc (3x5 mL) and the combined organic layers were dried over Na₂SO₄. The solvent was evaporated under reduced pressure and the residue was purified by flash chromatography (hexane) to yield the title compound (8 mg, 94 %) as a colourless oil. *R*_{f} = 0.32 (hexane); ¹H NMR (200 MHz, CDCl₃): *δ* = 7.72-7.65 (m, 4H), 7.46-7.33 (m, 6H), 6.03-5.87 (m, 1H), 5.73-5.61 (m, 1H), 5.11 (br s, 1H), 4.94 (br s, 1H), 4.75 (br s, 1H), 4.04-3.93 (m, 1H), 3.08 (dd, *J* = 16.1, 5.9 Hz, 1H), 2.84-2.70 (m, 2H), 2.19-1.44 (m, 8H), 1.41-1.17 (m, 6H), 1.09 (s, 9H), 0.88 (d, *J* = 5.8 Hz, 3H), 0.75 (d, *J* = 5.8 Hz, 3H); ¹³C NMR (50.3 MHz, CDCl₃): δ = 138.4, 135.9 (4C), 134.5 (2C), 133:8, 129.6, 129.5, 128.8, 128.6 (2C), 127.6, 127.5 (2C), 119.2, 111.1, 73.3, 39.4, 37.7, 37.3, 37.1, 36.3, 32.1, 30.7, 27.1 (3C), 26.6, 24.5, 23.1, 21.1 (2C), 19.3; IR (CCl₄): *ν* = 3070, 3018, 2955, 2925, 2856, 1470, 1459, 1423, 1385, 1366, 1308, 1108, 1071; [*α*]_{D}²⁰ =+68.4 (c = 0.25, EtOAc).

### Example 18

### (1R,4aR,6R,12aR)-1-Isopropyl-4-methyl-11-methylene-1,2,4a,5,6,7,10,11,12,12a-decahydro-benzocyclodecen-6-ol

The compound prepared in Example 17 (8 mg, 0.015 mmol) was dissolved in THF (1.0 mL) and TBAF (73 µL, 0.075 mmol, 1.0 M in THF) was added. The reaction mixture stirred 12 h at room temperature. Additional TBAF (73 µL, 0.075 mmol, 1.0 M in THF) was added and the reaction mixture stirred for another 12 h at room temperature. An aqueous phosphate buffer solution (1.0 mL, pH = 7) was added, the layers were separated and the aqueous layer was extracted with EtOAc (3x5 mL) and the combined organic layers were dried over Na₂SO₄. The solvent was evaporated under reduced pressure and the residue was purified by flash chromatography (hexanes/EtOAc 9:1) to yield the title compound (4 mg, quant.) as a colourless oil. *R*_{f} = 0.28 (hexanes/EtOAc 9:1); ¹H NMR (200 MHz, CDCl₃): *δ* = 5.76-5.62 (m, 2H), 5.26 (br s, 1H), 4.98 (s, 1H), 4.80 (s, 1H), 4.08-3.97 (m, 1H), 3.24-3.12 (m, 1H), 2.98-2.74 (m, 2H), 2.29-1.67 (m, 9H), 1.61-1.34 (m, 5H), 0.87 (d, *J =* 6.7 Hz, 4H), 0.77 (d, *J* = 6.7 Hz, 3H); ¹³C NMR (50.3 MHz, CDCl₃): *δ* = 133.8, 129.8, 127.0, 123.3, 120.0, 111.7, 72.0, 39.7, 37.7, 37.5, 36.9, 36.7, 32.2, 31.1, 26.8, 24.6, 23.6, 21.1 (2C); IR (CCl₄): *ν* = 3632, 3080, 3024, 2967, 2930, 1461, 1455, 1440, 1389, 1370; [α]_{D}²⁰ = +36.4 (c = 0.17, EtOAc).

### Example 19

### (E)-3-(1-Methyl-1H-imidazol-4-yl)-acrylic acid [(1R,4aR,6R,12aR)-(1-isopropyl-4-methyl-11-methylene-1,2,4a,5,6,7,10,11,12,12a-decahydro-benzocyclodecen-6-yl] ester

The compound prepared in Example 18 (4 mg, 0.0091 mmol) was dissolved in CH₂Cl₂ (1.0 mL) and added to Mixed Anhydride (51 mg, 0.137 mmol). NEt₃ (22 mg, 0.219 mmol) and DMAP (1 mg, 0.008 mmol) were added and the solution stirred for 3 d at room temperature. The solvent was evaporated under reduced pressure and the resulting residue was purified by flash chromatography (hexane/EtOAc 1:4) to yield the title compound (3 mg, 47 %) as a colourless oil. R_{*f*}= 0.30 (hexane/EtOAc 1:4); ¹H NMR (200 MHz, CDCl₃): δ = 7.62 (br s, 1H), 7.54 (d, *J =* 15.7 Hz, 1H), 7.08 (br s, 1H), 6.59 (d, *J =* 15.7 Hz, 1H), 5.74-5.62 (m, 2H), 5.30-5.11 (m, 2H), 5.04 (br s, 1H), 4.72 (br s, 1H), 3.73 (s, 3H), 3.25-2.73 (m, 3H), 2.34-1.55 (m, 7H), 1.44-1.21 (m, 6H), 0.87 (d, *J =* 6.7 Hz, 4H), 0.77 (d, *J =* 6.7 Hz, 3H);¹³C NMR (50.3 MHz, CDCl₃): *δ* = 164.3, 148.2, 138.2, 135.4, 131.5, 131.3, 129.6, 127.6, 119.7, 113.8, 111.6, 74.7, 39.3, 37.4, 36.9, 36.2, 34.8, 33.7, 30.9, 29.7, 29.5, 26.5, 24.5, 23.4, 21.0 (2C); IR (CCl₄): *ν* = 3380, 3075, 2956, 2922, 2856, 1766, 1709, 1645, 1460, 1428, 1382, 1368, 1305, 1159, 1099; [α]_{D}²⁰ = +6.0 (c = 0.05, EtOAc); HRMS (ESI): *m*/*z*: calcd for C₂₆H₃₇N₂O₂: 409.2855 [*M*+H]⁺; found: 409.2855.

### Examples 20 and 21

### (2S,3S)-1-{(1R,2R,6R)-2-[(2R)-2-(tert-Butyl-diphenyl-silanyloxy)-pent-4-enyl]-6-isopropyl-3-methyl-cyclohex-3-enyl}-3-methoxymethoxy-pent-4-en-2-ol (20) and (2R,3R)-1-{(1R,2R,6R)-2-[(2R)-2-(tert-Butyl-diphenyl-silanyloxy)-pent-4-enyl]-6-isopropyl-3-methyl-cyclohex-3-enyl}-3-metboxymethoxy-pent-4-en-2-ol (21)

Methoxymethyl allyl ether (180 mg, 1.75 mmol) in THF (4.0 mL) was cooled to -78 °C and *sec*-BuLi (1.10 mL, 1.45 mmol, 1.3 M in cyclohexane) was added. The reaction solution was stirred at -78 °C for 30 min and ¹Ipc₂BOMe (1.60 mL, 1.45 mmol, 0.93 M in THF) was then added. Stirring was maintained for 1 h, BF₃*Et₂O (263 mg, 1.86 mmol) was then added, followed by the compound prepared in example 7 (293 mg, 0.58 mmol) in THF (2.0 mL). The mixture was stirred at -78 °C for 5 h and then slowly warmed to room temperature. An aqueous NaOH solution (4.0 mL, 6.0 M) and H₂O₂ (4.0 mL, 35 %) were then added and the mixture left stirring overnight. H₂O (5 mL) was added and the aqueous layer was extracted with EtOAc (3x10 mL) and the combined organic layers were dried over Na₂SO₄. The solvent was evaporated under reduced pressure and the resulting residue was purified by flash chromatography (hexane/EtOAc 6:1) to provide unreacted starting compound (56 mg), the title compound 20 (246 mg, 70 %) and 21 (25 mg, 7 %) as colourless oils (total yield 77 %, 84 % after recovering starting material, de 82 %, **20:21** 10:1). **20**: *R*_{f} = 0.43 (hexane/EtOAc 6:1); ¹H NMR (200 MHz, CDCl₃): δ = 7.95-7.66 (m, 4H), 7.45-7.30 (m, 6H), 5.91-5.51 (m, 2H), 5.32-5.16 (m, 3H), 5.01-4.90 (m, 2H), 4.72 (d, *J* = 6.7 Hz, 1H), 4.56 (d, *J* = 6.7 Hz, 1H), 3.95 (q, *J* = 5.9 Hz, 1H), 3.79-3.72 (m, 1H), 3.58-3.47 (m, 1H), 3.38 (s, 3H), 2.35-2.14 (m, 5H), 1.93-1.14 (m, 11H), 1.05 (s, 9H), 0.79 (d, J= 6.6 Hz, 3H), 0.77 (d, J= 6.6 Hz, 3H); ¹³C NMR (50.3 MHz, CDCl₃): µ = 136.4, 135.9 (4C), 135.1, 134.9, 134.7, 134.6, 129.4 (2C), 127.4 (4C), 120.9, 119.9, 117.0, 93.9, 81.9, 72.2, 71.4, 55.7, 41.3, 39.3, 37.1, 35.8, 34.3, 31.3, 27.5, 27.1 (3C), 23.9, 22.9, 21.2, 19.3, 19.2; IR (CCl₄): *ν* = 3590, 3078, 2955, 2893, 2860, 1473, 1462, 1430, 1390, 1369, 1152, 1108, 935, 915; [α]_{D}²⁰ = +43.8 (c = 0.90, EtOAc); HRMS (ESI): *m*/*z:* calcd for C₃₈H₅₆NaO₄Si: 627.3845 [*M*+Na]⁺; found: 627.3828; **21:** *R*_{f} = 0.37 (hexane/EtOAc 6:1); ¹H NMR (200 MHz, CDCl₃): δ = 7.71-7.60 (m, 4H), 7.47-7.30 (m, 6H), 5.96-5.77 (m, 1H), 5.65-5.49 (m, 1H), 5.38-5.21 (m, 2H), 5.16 (br s, 1H), 5.07-4.92 (m, 2H), 4.73 (d, *J* = 6.7 Hz, 1H), 4.58 (d, *J* = 6.7 Hz, 1H), 3.98-3.83 (m, 1H), 3.81-3.69 (m, 1H), 3.65-3.50 (m, 1H), 3.39 (s, 3H), 2.46-1.95 (m, 4H), 1.90-0.98 (m, 20H), 0.90-0.87 (m, 1H), 0.83 (d, *J* = 6.8 Hz, 3H), 0.69 (d, *J* = 6.8 Hz, 3H); ¹³C NMR (50.3 MHz, CDCl₃): *δ* = 137.3, 135.9 (4C), 135.1, 134.8, 134.7 (2C), 129.4 (2C), 127.4 (4C), 121.9, 120.2, 117.0, 93.7, 82.4, 72.1, 70.3, 55.8, 40.8, 37.5, 37.1, 35.8, 35.4, 31.3, 29.7, 27.0 (3C), 26.8, 24.3, 23.9, 21.1, 19.3; IR (CCl₄): *ν* = 3600, 3080, 2960, 2938, 2899, 2862, 1475, 1432, 1391, 1372, 1158, 1108, 912; [α]_{D}²⁰ =+48.1 (c = 1.09, EtOAc); HRMS (ESI): *m*/*z:* calcd for C₃₈H₅₆NaO₄Si: 627.3846 [*M*+Na]⁺; found: 627.3860.

### Example 22

### 2,2-Dimethyl-propionic acid {(1S,2S)-1-{(1R,2R,6R)-2-[(2R)-2-(tert-butyl-diphenyl-silanyloxy)-pent-4-enyl]-6-isopropyl-3-methyl-cyclohex-3-enylmethyl}-2-methoxymethoxy-but-3-enyl} ester.

The compound prepared in Example 20 (173 mg, 0.29 mmol) was dissolved in pyridine (2.0 mL). DMAP (4 mg, 0.03 mmol) and PivCl (172 mg, 1.43 mmol) were added. The mixture stirred at room temperature for 72 h. EtOAc (10 mL) were added, the organic layer was washed with a saturated aqueous KHSO₄ solution (2x10 mL), the combined aqueous layers were back extracted with EtOAc (3x10 mL) and the combined organic layers were dried over Na₂SO₄ The solvent was evaporated under reduced pressure and the resulting residue was purified by flash chromatography (hexane/EtOAc 9:1) to provide the title compound (158 mg, 80 %) as a colourless oil. R_{f} = 0.52 (hexane/EtOAc 9:1); ¹H NMR (300 MHz, CDCl₃): δ = 7.71-7.66 (m, 4H), 7.45-7.28 (m, 6H), 5.85-5.59 (m, 2H), 5.29-5.17 (m, 3H), 5.03-4.90 (m, 3H), 4.65 (d, *J =* 6.3 Hz, 1H), 4.56 (d, *J* = 6.3 Hz, 1H), 4.09-3.87 (m, 2H), 3.35 (s, 3H), 2.30-2.12 (m, 3H), 1.85 (br s, 1H), 1.72-1.22 (m, 11H), 1.18 (s, 9H), 1.04 (s, 9H), 0.82 (d, *J* = 6.3 Hz, 3H), 0.72 (d, *J* = 6.3 Hz, 3H); ¹³C NMR (50.3 MHz, CDCl₃): *δ* = 177.6, 135.9 (4C), 135.3, 134.8, 134.6 (2C), 134.1, 129.5 (2C), 127.5 (4C), 121.0, 119.0, 117.2, 94.4, 77.8, 72.2, 71.7, 55.6, 41.4, 39.4, 36.8, 34.8, 32.7, 27.4, 27.3 (3C), 27.2, 27.0 (3C), 23.6, 22.5, 21.0, 20.6, 19.3 (2C); IR (CCl₄): *ν* = 3061, 2942, 2920, 2882, 2842, 1722, 1455, 1467, 1458, 1423, 1392, 1385, 1362, 1150, 1107, 1098, 909; [α]_{D}²⁰ = +25.5 (c = 0.78, EtOAc); HRMS (ESI): *m*/*z*: calcd for C₄₃H₆₄NaO₅Si: 711.4420 [M+Na]⁺; found: 711.4412.

### Example 23

### 2,2-Dimethyl-propionic acid {(1S,2S)-1-{(1R,2R,6R)-2-[(2R)-2-(tert-butyl-diphenyl-silanoxy)-pent-4-enyl]-6-isopropyl-3-methyl-cyclohex-3-enylmethyl}-2-hydroxy-but-3-enyl} ester.

The compound prepared in Example 22 (10 mg, 0.015 mmol) was dissolved in CH₂Cl₂ (0.3 mL) and cooled to -78 °C. PhSH (2 mg, 0.015 mmol) and BF₃*Et₂O (4 mg, 0.029 mmol) were added and the solution was allowed to warm to -10 °C and stirred at -10 °C for 2 h. Then a saturated aqueous NaHCO₃ solution (2 mL) was added and the solution was warmed up to room temperature. The layers were separated, the aqueous layer was extracted with EtOAc (3x5 mL) and the combined organic layers were dried over Na₂SO₄. The solvent was evaporated under reduced pressure and the resulting residue was purified by flash chromatography (hexane/EtOAc 8:2) to provide the title compound (6 mg, 64 %) as a colourless oil. *R*_{f} = 0.54 (hexane/EtOAc 8:2); ¹H NMR (200 MHz, CDCl₃): δ = 7.71-7.67 (m, 4H), 7.41-7.31 (m, 6H), 5.89-5.72 (m, 2H), 5.34 (br s, 1H), 5.25-5.14 (m, 2H), 5.03-4.91 (m, 3H), 4.08 (br s, 1H), 3.93-3.87 (m, 1H), 2.27-2.16 (m, 3H), 2.01-1.22 (m, 14H), 1.18 (s, 9H), 1.04 (s, 9H), 0.93-0.88 (m, 1H), 0.82 (d, *J =* 6.4 Hz, 3H), 0.72 (d, *J =* 6.4 Hz, 3H); ¹³C NMR (50.3 MHz, CDCl₃): δ = 176.1, 137.1 (2C), 136.4, 135.9 (2C), 135.0 (2C), 134.7, 129.5 (2C), 127.4 (4C), 121.0, 117.2, 116.4, 74.4, 73.4, 72.3, 41.4, 39.2, 36.7, 35.0, 33.3, 28.0, 27.5, 27.3 (3C), 27.0 (3C), 23.8, 22.6, 20.9 (2C), 20.0, 19.3 (2C); IR (CCl₄): *ν* = 3618, 3582, 3060, 2942, 2918, 2842, 1724, 1472, 1465, 1458, 1421, 1382, 1363, 1149, 1101, 1060, 908; [α]_{D}²⁰ = +6.1 (c = 0.56, EtOAc); HRMS (ESI): *m*/*z*: calcd for C₄₁H₆₂NaO₄Si: 669.4315 [*M*+Na]⁺; found: 669.4299.

### Example 24

### 2,2-Dimethyl-propionic acid [(4R,4aR,6S,7S,11R,12aR)-11-(tert-butyl-diphenyl-silanoxy)-7-hydrox-y4-isopropyl-1-methyl-3,4,4a,56,7,10,11,12,12a-decahydro-benzocyclodecen-6-yl] ester

The compound prepared in Example 23 (33 mg, 0.05 mmol) was dissolved in degassed CH₂Cl₂ (5.1 mL). Grubbs-II (2 mg, 2.5 µmol) in degassed CH₂Cl₂ (0.5 mL) was slowly added. The reaction mixture stirred for 2 d at room temperature. Additional Grubbs-II (1 mg, 1.3 µmol) in degassed CH₂Cl₂ (0.2 mL) was slowly added and the mixture stirred for further 2 d at room temperature. Additional Grubbs-II (1 mg, 1.3 µmol) in degassed CH₂Cl₂ (0.2 mL) was slowly added and the mixture stirred for further 1 d at room temperature. The solvent was evaporated under reduced pressure and the residue was purified by flash chromatography (hexane/EtOAc 8:2) to provide the title compound (23 mg, 73 %) as a colourless oil. R_{*f*}*=* 0.44 (hexane/EtOAc 8:2); ¹H NMR (400 MHz, CDCl₃): δ = 7.73-7.65 (m, 4H), 7.47-7.35 (m, 6H), 5.99 (dt, J= 11.5, 5.2 Hz, 1H), 5.58 (t, J= 10.5 Hz, 1H), 5.14 (br s, 1H), 5.05-4.96 (m, 1H), 4.77 (t, J= 9.9 Hz, 1H), 4.23-4.16 (m, 1H), 2.65-2.57 (m, 1H), 2.32-2.27 (m, 1H), 1.89-1.54 (m, 9H), 1.40-1.05 (m, 23H), 0.84 (d, *J* = 6.8 Hz, 3H), 0.64 (d, J= 6.8 Hz, 3H); ¹³C NMR (50.3 MHz, CDCl₃): δ = 178.1, 137.1, 135.8 (4C), 135.4, 134.8, 134.5 (2C), 129.4 (2C), 127.4 (4C), 121.0, 117.2, 116.4, 74.4, 73.3, 72.2, 41.3, 39.2, 36.6, 34.6, 33.1, 27.7, 27.3 (3C), 26.9 (3C), 23.6, 22.6, 20.9 (2C), 20.0; IR (CCl₄): *ν*= 3630, 2958, 2925, 2852, 1730, 1425, 1367, 1155, 1112, 908; [α]_{D}²⁰ = +17.5 (c = 0.60, EtOAc); HRMS (ESI): *m*/*z:* calcd for C₃₉H₅₆NaO₄Si: 639.3846 [*M*+Na]⁺; found: 639.3855.

### Example 25

### 2,2-Dimethyl-propionic acid [(4R,4aR,6S,7S,11R,12aR)-11-(tert-butyl-diphenyl-silanyloxy)-4-isopropyl-7-methoxy-1-methyl-3,4,4a,5,6,7,10,11,12,12a-decahydro-benzocyclodecen-6-yl] ester

The compound prepared in Example 24 (14 mg, 0.023 mmol) was dissolved in CH₂Cl₂ (0.2 mL). 2,6-Di-*tert*-butyl-pyridine (44 mg, 0.23 mmol) and MeOTf (19 mg, 0.12 mmol) were added and the solution stirred for 18 h at 40 °C. The solvent was evaporated under reduced pressure and the resulting residue was purified by flash chromatography (hexane/EtOAc 9:1) to provide the title compound (14 mg, 96 %) as a colourless oil. *R*_{*f*} = 0.28 (hexane/EtOAc 9:1); ¹H NMR (200 MHz, CDCl₃): δ = 7.74-7.60 (m, 4H), 7.54-7.27 (m, 6H), 6.08 (dt, *J =* 11.4, 5.3 Hz, 1H), 5.40 (t, *J =* 10.7 Hz, 1H), 5.16-5.01 (m, 2H), 4.29-4.08 (m, 2H), 3.18 (s, 3H), 2.68-2.49 (m, 1H), 2.35-2.17 (m, 1H), 2.09-0.84 (m, 31H), 0.80 (d, *J=* 6.8 Hz, 3H), 0.57 (d, *J=* 6.8 Hz, 3H); ¹³C NMR (75.5 MHz, CDCl₃): δ = 177.7, 138.6, 135.8 (4C), 134.3 (2C), 130.9, 129.6 (2C), 129.2, 127.6 (4C), 120.5, 78.8, 74.8, 72.2, 56.2, 37.6, 37.1, 36.4, 35.3, 32.6, 29.7, 27.2 (3C), 27.0 (3C), 26.9, 26.7, 24.3, 24.1, 21.0 (2C), 19.2; IR (CCl₄): *ν* = 2948, 2920, 2848, 1723, 1475, 1422, 1385, 1363, 1279, 1155, 1099, 1059; [*α*]_{D}²⁰ = +7.8 (c = 0.77, EtOAc); HRMS (ESI): *m*/*z*: calcd for C₄₀H₅₈NaO₄Si: 653.4002 [M+Na]⁺; found: 653.3986.

### Example 26

### 2,2-Dimethyl-propionic acid [(4R,4aR,6S,7S,11R,12aR)-11-hydroxy-4-isopropyl-7-methoxy-1-methyl-3,4,4a,5,6,7,10,11,12,12a-decahydro-benzocyclodecen-6-yl] ester.

The compound prepared in Example 25 (14 mg, 0.022 mmol) was dissolved in THF (0.50 mL) and TBAF (0.044 mL, 0.044 mmol, 1.0 M in THF) was added. The reaction mixture stirred 14 h at room temperature. An aqueous phosphate buffer solution (1.0 mL, pH = 7) was added, the layers were separated and the aqueous layer was extracted with EtOAc (3x5 mL) and the combined organic layers were dried over Na₂SO₄. The solvent was evaporated under reduced pressure and the residue was purified by flash chromatography (hexanes/EtOAc 8:2) to yield the title compound (8 mg, 89 %) as a colourless oil. *R*_{f} = 0.11 (hexanes/EtOAc 8:2); ¹H NMR (400 MHz, CDCl₃): δ = 5.87 (dt, *J =* 11.6, 5.4 Hz, 1H), 5.44 (t, *J*= 10.8 Hz, 1H), 5.32 (br s, 1H), 5.12-5.08 (m, 1H), 4.34-4.23 (m, 2H), 3.26 (s, 3H), 2.80-2.74 (m, 1H), 2.48-2.2.31 (m, 1H), 2.25-1.12 (m, 20H), 1.05-0.89 (m, 3H), 0.86 (d, *J =* 6.8 Hz, 3H), 0.64 (d, *J =* 6.8 Hz, 3H); ¹³C NMR (100.8 MHz, CDCl₃): δ= 177.6, 137.5, 130.0, 129.5, 121.0, 78.3, 74.7, 70.7, 56.2, 37.8, 37.3, 36.9, 36.6, 35.3, 32.4, 29.6, 27.2 (3C), 26.9, 26.7, 24.4, 21.0 (2C); IR (CCl₄): *ν* = 3620, 2954, 2923, 2864, 1728, 1478, 1451, 1395, 1386, 1367, 1280, 1160; 1099, 905; [*α*]_{D}²⁰ =-8.4 (c = 0.37, EtOAc).

### Example 27

### (E)-3-(1-Methyl-1H-imidazol-4-yl)-acrylic acid [(1R,4aR,6R,10S,11S,12aR)-11-(2,2-dimethyl-propionyloxy)-1-isopropyl-10-methoxy-4-methyl-1,2,4a,5,6,7,10,11,12,12a-decahydro-benzocyclodecen-6-yl] ester

The compound prepared in Example 26 (6 mg, 0.0153 mmol) was dissolved in CH₂Cl₂ (1.8 mL) and added to Mixed Anhydride (76 mg, 0.325 mmol). NEt₃ (33 mg, 0.325 mmol) and DMAP (2 mg, 0.0153 mmol) were added and the solution stirred for 15 d at room temperature. The solvent was evaporated under reduced pressure and the resulting residue was purified by flash chromatography (hexane/EtOAc 1:5) to yield the title compound (6 mg, 66 %) as a colourless oil. R_{*f*} = 0.22 (hexane/EtOAc 1:5); ¹H NMR (400 MHz, CDCl₃): *δ =* 7.56 (d, *J =* 15.6 Hz, 1H), 7.48 (s, 1H), 7.10 (s, 1H), 6.53 (d, *J =* 15.6 Hz, 1H), 5.96-5.89 (m, 1H), 5.52-5.38 (m, 2H), 5.32 (br s, 1H), 5.12 (d, *J =* 7.3 Hz, 1H), 4.27 (t, *J =* 9.9 Hz, 1H), 3.73 (s, 3H), 3.26 (s, 3H), 2.88-2.76 (m, 1H), 2.54-2.46 (m, 1H), 2.13-1.14 (m, 24H), 0.99-0.87 (m, 2H), 0.85 (d, *J =* 5.1 Hz, 3H), 0.65 (d, *J =* 5.1 Hz, 3H);¹³C NMR (100.8 MHz, CDCl₃): *δ* = 177.7, 166.6, 139.2, 138.5, 136.1, 135.7, 130.3, 129.6, 122.3, 121.1, 116.3, 79.7, 74.7, 72.9, 56.7, 37.4 (2C), 35.3, 33.5, 33.2, 30.7, 29.6, 29.3, 27.2 (3C), 26.9, 26.6, 24.3, 24.4, 21.0 (2C); IR (CCl₄): *ν* = 2960, 2931, 2889, 2861, 1731, 1712, 1645, 1481, 1460, 1389, 1300, 1157, 1103; [α]_{D}²⁰ = -17.0 (c = 0.33, EtOAc); HRMS (ESI): *m*/*z:* calcd for C₃₂H₅₀NaN₂O₅: 565.3617 [*M*+Na]⁺; found: 565.3611.

### Example 28

### 2,2-Dimethyl-propionic acid [(4R,4aR,6S,7S, 11R,12aR)-7-acetoxy-11-(tert-butyl-diphenyl-silanyloxy)-4-isopropyl-1-methyl-3,4,4a,5,6,7,10,11,12,12a-decahydro-benzocyclodecen-6-yl] ester

The compound prepared in Example 24 (16 mg, 0.026 mmol) was dissolved in pyridine (0.5 mL). Ac₂O (5 mg, 0.052 mmol) and DMAP (cat.) were added and the reaction mixture stirred for 24 h at room temperature. EtOAc (5 mL) was added and the organic layer was washed with a saturated aqueous KHSO₄ solution (2x5 mL), the aqueous layer was back extracted with EtOAc (3x5 mL) and the combined organic layers were dried over Na₂SO₄. The solvent was evaporated under reduced pressure and the residue was purified by flash chromatography (hexane/EtOAc 9:1) to provide the title compound (12 mg, 71 %) as a colourless oil. *R*_{f} *=* 0.69 (hexane/EtOAc 9:1); ¹H NMR (200 MHz, CDCl₃): *δ* = 7.69-7.61 (m, 4H), 7.46-7.30 (m, 6H), 6.11-5.87 (m, 2H), 5.45 (t, J= 10.7 Hz, 1H), 5.22-5.10 (m, 2H), 4.24-4.18 (m, 1H), 2.88-2.74 (m, 1H), 2.32-2.25 (m, 1H), 2.12-1.95 (m, 4H), 1.84-0.89 (m, 30H), 0.82 (d, *J* = 6.9 Hz, 3H), 0.57 (d, *J* = 6.9 Hz, 3H); ¹³C NMR (75.5 MHz, CDCl₃): *δ* = 177.5, 170.0, 138.8, 135.7 (4C), 134.1 (2C), 132.1, 129.6,129.5,127.6 (2C), 127.5 (2C), 126.5, 120.3, 73.6, 72.0, 71.5, 37.7, 37.3, 36.4, 35.3, 32.9, 29.6, 27.04 (7C), 26.5, 24.3, 24.0 (2C), 21.0, 19.2, 14.5; IR (CCl₄): *ν*= 3078, 2959, 2938, 2860, 1739, 1732, 1482, 1460, 1430, 1371, 1155, 1112, 1070; [*α*]²⁰_{D} +0.6 (c = 0.51, EtOAc); HRMS (ESI): *m*/*z*: calcd for C₄₁H₅₈NaO₅Si: 681.3951 [*M*+Na]⁺; found: 681.3967.

### Example 29

### 2,2-Dimethyl-propionic acid [(4R,4aR,6S,7S,11R,12aR)-7-acetoxy-11-hydroxy-4-isopropyl-1-methyl-3,4,4a,5,6,7,10,11,12,12a-decahydro-benzocyclodecen-6-yl] ester.

The compound prepared in Example 28 (11 mg, 0.0167 mmol) was dissolved in THF (0.30 mL) and TBAF (0.033 mL, 0.0334 mmol, 1.0 M in THF) was added. The reaction mixture stirred 20 h at room temperature. An aqueous phosphate buffer solution (1.0 mL, pH = 7) was added, the layers were separated and the aqueous layer was extracted with EtOAc (3x5 mL) and the combined organic layers were dried over Na₂SO₄. The solvent was evaporated under reduced pressure and the residue was purified by flash chromatography (hexanes/EtOAc 8:2) to yield the title compound (7 mg, 92 %) as a colourless oil. *R*_{f} = 0.15 (hexanes/EtOAc 8:2); ¹H NMR (200 MHz, CDCl₃): *δ* = 6.01 (t, *J =* 10.3 Hz, 1H), 5.91 (dt, *J =* 11.4, 5.4 Hz, 1H), 5.47 (t, *J =* 10.7 Hz, 1H), 5.29 (br s, 1H), 5.19 (dd, *J =* 10.1, 6.9 Hz, 1H), 4.46-4.24 (m, 1H), 3.08-2.91 (m, 1H), 2.44-2.33 (m, 1H), 2.18-1.22 (m, 17H), 1.18 (s, 9H), 0.84 *(d, J =* 6.8 Hz, 3H), 0.61 (d, *J =* 6.8 Hz, 3H); ¹³C NMR (75.5 MHz, CDCl₃): δ = 177.4, 171.1, 138.8, 130.9, 127.3, 120.9, 73.5, 71.3, 70.5, 37.9, 37.4, 36.6, 35.3, 32.7, 29.7, 27.1 (3C), 26.9, 26.4, 24.4, 21.0 (2C), 14.5; IR (CCl₄): *ν* = 3622, 2957, 2935, 2870, 1745, 1730, 1479, 1452, 1395, 1388, 1369, 1280, 1155; [*α*]_{D}²⁰ = -33.5 (c = 0.39, EtOAc); HRMS (ESI): *m*/*z:* calcd for C₂₅H₄₀NaO₅: 443.2773 [M+Na]⁺; found: 443.2761.

### Example 30

### (E)-3-(1-Methyl-1H-imidazol-4-yl)-acrylic acid [(1R,4aR,6R,10S,11S,12aR)-10-acetoxy-11-(2,2-dimethyl-propionyloxy)-1-isopropyl-4-methyl-1,2,4a,5,6,7,10,11,12,12a-decahydro-benzocyclodecen-6-yl] ester

The compound prepared in Example 29 (6 mg, 0.014 mmol) was dissolved in CH₂Cl₂ (1.6 mL) and added to Mixed Anhydride (50 mg, 0.21 mmol). NEt₃ (21 mg, 0.21 mmol) and DMAP (2 mg, 0.014 mmol) were added and the solution stirred for 2 d at room temperature. The solvent was evaporated under reduced pressure and the resulting residue was purified by flash chromatography (hexane/EtOAc 1:4) to yield the title compound (6 mg, 80 %) as a colourless oil. R_{*f*}= 0.20 (hexane/EtOAc 1:4); ¹H NMR (400 MHz, CDCl₃): *δ =* 7.60 (s, 1H), 7.54 (d, *J* = 15.7 Hz, 1H), 7.11 (s, 1H), 6.59 *(d, J =* 15.7 Hz, 1H), 6.04 (t, *J* 10.4 Hz, 1H), 5.89 (dt, *J=*11.6*,* 5.6 Hz, 1H), 5.55-5.45 (m, 2H), 5.32 (s, 1H), 5.23 (dd, *J* = 10.1, 6.8 Hz, 1H), 3.75 (s, 3H), 3.13-3.03 (m, 1H), 2.54-2.48 (m, 1H), 2.22-1.17 (m, 25H), 0.85 (d, *J =* 6.9 Hz, 3H), 0.63 (d, *J =* 6.9 Hz, 3H); ¹³C NMR (75.5 MHz, CDCl₃):*δ* = 177.6, 170.1, 166.7, 138.6, 138.4, 136.1, 130.9, 127.5 (2C), 122.3, 120.9, 116.3, 73.4, 72.8, 71.1, 37.5, 36.5, 35.3, 33.7, 33.5, 30.0, 29.7, 27.2 (3C), 26.8, 26.3, 24.5, 21.0 (3C), 14.5; IR (CC4): *ν* = 2961, 2935, 1742, 1733, 1712, 1648, 1390, 1371, 1155; [*α*]_{D}²⁰= - 39.6 (c = 0.24, EtOAc); HRMS (ESI): *m*/*z:* calcd for C₃₂H₄₇N₂O₆: 555.3434 [M+H]⁺; found: 555.3425.

### Example 31

### 2,2-Dimethyl-propionic acid {(4R,4aR,6S,7S,11R,12aR)-11-(tert-butyl-diphenyl-silanyloxy)-4-isopropyl-1-methyl-7-[(2'*)-tetrahydro-pyran-2'-yloxy)]-3,4,4a,5,6,7,10,11,12,12a-decahydro-benzocyclodecen-6-yl} ester.

The compound prepared in Example 24 (29 mg, 0.047 mmol) was dissolved in CH₂Cl₂ (0.4 mL). Dihydropyran (6 mg, 0.071 mmol) and PPTS (1 mg, 0.005 mmol) were added and the solution stirred for 11 h at room temperature. *i*-Pr₂O (5 mL) was added, the organic layer was washed with a semisaturated NaCl solution (2x5 mL) and the organic layer was dried over Na₂SO₄. The solvent was evaporated under reduced pressure and the resulting residue was purified by flash chromatography (hexane/EtOAc 14:1) to yield the title compound (25 mg, 77 %) as a colourless oil. R_{*f*} = 0.33 (hexane/EtOAc 14:1); ¹H NMR (250 MHz, CDCl₃, signal doubling due to diastereomers): δ= 7.68-7.61 (m, 4H), 7.38-7.28 (m, 6H), 6.08+5.93 (dt, *J =* 11.6, 5.5 Hz, 1H), 5.59+5.32 (t, *J* = 10.4 Hz, 1H), 5.18-5.01 (m, 2H), 4.88-4.61 (m, 2H), 4.23-4.10 (m, 1H), 3.94-3.72 (m, 1H), 3.51-3.33 (m, 1H), 2.76-2.57 (m, 1H), 2.35-2.17 (m, 1H), 2.13-1.95 (m, 1H), 1.91-0.95 (m, 36H), 0.80 (d, *J=* 5.8 Hz, 3H), 0.61-0.55 (m, 3H); ¹³C NMR (50.3 MHz, CDCl₃, signal doubling due to diastereomers): *δ* = 177.6+177.4, 138.9+138.8, 135.7 (4C), 134.4+134.2 (2C), 132.0, 130.0+129.5 (2C), 128.4, 127.9+127.5+127.4 (4C), 120.2, 99.2+93.0, 76.5, 74.9+74.6, 72.1+70.5, 61.5+60.4, 37.7, 37.3, 36.5, 35.4, 32.6, 30.4+30.1, 29.6, 27.3 (3C), 27.0+26.6 (3C), 26.3, 25.4, 24.3, 23.9, 21.0 (2C), 19.2, 16.7+16.3, 14.6; IR (CCl₄): *ν* = 3062, 2945, 2918, 2844, 1722, 1474, 1465, 1448, 1422, 1381, 1362, 1151, 1107, 1059, 902; [*α*]_{D}²⁰ =-7.2 (c = 1.16, EtOAc); HRMS (ESI): *m*/*z:* calcd for C₄₄H₆₄NaO₅Si: 723.4421 [*M*+Na]⁺; found: 723.4416.

### Example 32

### 2,2-Dimethyl-propionic acid {(4R,4aR,6S,7S, 11R, 12aR)-11-hydroxy-4-isopropyl-1-methyl-[(2'*)-7-(tetrahydro-pyran-2'-yloxy)]-3,4,4a,5,6,7,10,11,12,12a-decahydro-benzocyclodecen-6-yl} ester.

The compound prepared in Example 31 (23 mg, 0.033 mmol) was dissolved in THF (2.0 mL) and TBAF (165 µL, 0.165 mmol, 1.0 M in THF) was added. The reaction mixture stirred 20 h at room temperature. An aqueous phosphate buffer solution (1.0 mL, pH = 7) was added, the layers were separated and the aqueous layer was extracted with EtOAc (3x5 mL) and the combined organic layers were dried over Na₂SO₄. The solvent was evaporated under reduced pressure and the residue was purified by flash chromatography (hexanes/EtOAc 4:1) to yield the title compund (15 mg, quant.) as a colourless oil. *R*_{*f*} = 0.10 (hexanes/EtOAc 4:1); ¹H NMR (200 MHz, CDCl₃, signal doubling due to diastereomers): *δ* = 5.97+5.78 (dt, *J =* 9.3, 5.8 Hz, 1H), 5.60+5.35 (t, *J =* 10.3 Hz, 1H), 5.29-5.01 (m, 2H), 4.93-4.65 (m, 2H), 4.39-4.15 (m, 1H), 4.02-3.73 (m, 1H), 3.55-3.33 (m, 1H), 2.93-2.71 (m, 1H), 2.40-2.22 (m, 1H), 2.17-1.09 (m, 29H), 0.82 (d, *J* = 6.7 Hz, 3H), 0.62+0.59 (d, *J =* 6.7 Hz, 3H); ¹³C NMR (100.8 MHz, CDCl₃, signal doubling due to diastereomers): *δ* = 177.5, 133.8, 129.2, 126.6, 120.9+120.8, 99.3+93.1, 76.5, 74.9+74.7, 70.7+70.4, 61.5+60.6, 37.9+37.8, 37.4, 36.6, 35.5, 32.4, 30.4+30.2, 27.4, 27.3 (3C), 27.2, 26.9, 26.7+26.3, 25.5+25.4, 24.5, 21.1 (2C), 18.7+18.4; IR (CCl₄): *ν* = 3620, 3440, 2950, 2865, 1723, 1477, 1451, 1392, 1385, 1365, 1279, 1155, 1110, 1072, 1050, 905; [*α*]_{D}²⁰ = - 36.1 (c = 0.75, EtOAc); HRMS (ESI): *m*/*z:* calcd for C₂₈H₄₆NaO₅: 485.3243 [*M*+Na]⁺; found: 485.3238.

### Example 33

### 3-(1-Methyl-1H-imidazol-4-yl)-acrylic acid {(1R,4aR,6R,10S,11S,12aR)-11-(2,2-dimethyl-propionyloxy)-1-isopropyl-4-methyl-10-[(2'*)-(tetrahydro-pyran-2-yloxy)]-1,2,4a,5,6,7,10,11,12,12a-decahydro-benzocyclodecen-6-yl} ester.

The compound prepared in Example 32 (15 mg, 0.032 mmol) was dissolved in CH₂Cl₂ (2.0 mL) and added to Mixed Anhydride (118 mg, 0.494 mmol). NEt₃ (50 mg, 0.494 mmol) and DMAP (4 mg, 0.032 mmol) were added and the solution stirred for 3 d at room temperature. The solvent was evaporated under reduced pressure and the resulting residue was purified by flash chromatography (hexane/EtOAc 1:4) to yield the title compound (12 mg, 61 %) as a colourless oil. R_{*f*} = 0.15 (hexane/EtOAc 1:4); ¹H NMR (400 MHz, CDCl₃, signal doubling due to diastereomers): *δ* = 7.62 (s, 1H), 7.57+7.53 (d, *J* = 15.7 Hz, 1H), 7.10 (s, 1H), 6.56 (d, *J* = 15.7 Hz, 1H), 5.94+5.75 (dt, *J =* 9.1, 6.2 Hz, 1H), 5.65 (t, *J =* 10.4 Hz, 1H), 5.45-5.37 (m, 2H), 5.30 (s, 1H), 5.23-5.08 (m, 1H), 4.92+4.78 (t, *J* = 10.2 Hz, 1H), 4.90-4.85+4.68-4.65 (m, 1H), 3.99-3.92+3.84-3.77 (m, 1H), 3.74 (s, 3H), 3.57-3.47+3.45-3.38 (m, 1H), 2.97-2.83 (m, 1H), 2.48-2.41 (m, 1H), 2.17-1.38 (m, 15H), 1.33-1.18 (m, 12H), 0.84 (d, J= 6.8 Hz, 3H), 0.67-0.61 (m, 3H); ¹³C NMR (50.3 MHz, CDCl₃, signal doubling due to diastereomers): *δ* = 177.2, 166.5, 137.9, 135.2, 130.9+130.8, 129.4, 126.7, 122.1, 120.8, 117.0, 99.3+93.3, 74.7+74.5, 73.1, 70.2, 61.5+60.8, 37.4, 36.6, 35.4, 33.7, 30.3, 29.6, 27.3 (3C), 27.0, 26.8, 26.5, 26.2, 25.4, 24.5, 24.3, 21.0 (2C), 19.0+18.7, 14.6; IR (CCl₄): ν = 2959, 2877, 1725, 1709, 1645, 1481, 1452, 1385, 1398, 1156, 1113, 909; [α]_{D}²⁰ = -44.4 (c = 0.59, EtOAc); HRMS (ESI): *m*/*z*: calcd for C₃₂H₅₃N₂O₆: 597.3904 [M+H]⁺; found: 597.3900.

### Example 34

### 3-(1-Methyl-1H-imidazol-4-yl)-acrylic acid [(1R,4aR,6R,10S,11S,12aR)-11-(2,2-dimethyl-propionyloxy)-10-hydroxy-1-isopropyl-4-methyl-1,2,4a,5,6,7,10,11,12,12a-decahydro-benzocyclodecen-6-yl] ester.

The compound prepared in Example 33 (13 mg, 0.022 mmol) was dissolved in EtOH (2.0 mL, 80 %). PTSA (0.8 mg, 0.0044 mmol) were added and the solution stirred for 72 h at room temperature. EtOAc (5 mL) was added, the organic layer was washed with a saturated aqueous NaHCO₃ solution (2x5 mL), with a saturated aqueous NaCl solution (5 mL) and then the organic layer was dried over Na₂SO₄. The solvent was evaporated under reduced pressure and the resulting residue was purified by flash chromatography (EtOAc) to yield the title compound (7 mg, 82 %) as a colourless oil. R_{*f*}= 0.16 (EtOAc); ¹H NMR (400 MHz, CDCl₃): *δ*= 7.67 (s, 1H), 7.54 (d, *J =* 5.2 Hz, 1H), 7.09 (s, 1H), 6.58 (d, *J=* 5.2 Hz, 1H), 5.77 (dt, *J =* 11. 5, 5.3 Hz, 1H), 5.60 (t, *J =* 10.5 Hz, 1H), 5.43-5.37 (m, 1H), 5.29 (s, 1H), 5.11-5.00 (m, 1H), 4.84 (t, *J* = 9.8 Hz, 1H), 3.74 (s, 3H), 2.87-2.78 (m, 1H), 2.51-1.71 (m, 11H), 1.62-1.36 (m, 3H), 1.35-1.15 (m, 10H), 0.84 (d, *J*= 6.8 Hz, 3H), 0.64 (d, *J* = 6.8 Hz, 3H); ¹³C NMR (100.8 MHz, CDCl₃): *δ* = 178.5, 166.5, 138.9, 137.7, 135.1, 131.4, 128.2, 122.4, 121.1, 117.2, 77.7, 73.1, 70.4, 69.5, 37.4, 33.9, 33.1, 30.1, 29.7, 27.3 (3C), 27.2, 26.9, 26.8, 24.4, 24.3, 21.0 (2C), 19.1; IR (CCl₄): *ν* = 3620, 3240, 2957, 2922, 2865, 1709, 1643, 1478, 1455, 1385, 1367, 1322, 1297, 1155, 1109, 1043, 908; [α]_{D}²⁰ =-45.0 (c = 0.28, EtOAc); HRMS (ESI): *m*/*z:* calcd for C₃₀H₄₅N₂O₅: 513.3329 [*M*+H]⁺; found: 513.3321.

### Example 35

### 2,2-Dimethyl-propionic acid {(1R,2R)-1-{(1R,2R,6R)-2-[(2R)-2-(tert-butyl-diphenyl-silanyloxy)-pent-4-enyl]-6-isopropyl-3-methyl-cyclohex-3-enylmethyl}-2-methoxymethoxy-but-3-enyl} ester.

The compound prepared in Example 21 (26 mg, 0.043 mmol) was dissolved in pyridine (1.0 mL). DMAP (1 mg, 0.004 mmol) and PivCl (26 mg, 0.215 mmol) were added. The mixture stirred at room temperature for 72 h. EtOAc (5 mL) were added, the organic layer was washed with a saturated aqueous KHSO₄ solution (2x5 mL), the combined aqueous layers were back extracted with EtOAc (3x5 mL) and the combined organic layers were dried over Na₂SO₄ The solvent was evaporated under reduced pressure and the resulting residue was purified by flash chromatography (hexane/EtOAc 9:1) to provide the title compound (27 mg, 91 %) as a colourless oil. R_{*f*} = 0.71 (hexane/EtOAc 9:1); ¹H NMR (200 MHz, CDCl₃): *δ* = 7.67-7.59 (m, 4H), 7.42-7.28 (m, 6H), 6.03-5.91 (m, 1H), 5.87-5.76 (m, 1H), 5.31-4.95 (m, 6H), 4.63 (d, *J* = 6.7 Hz, 1H), 4.55 (d, *J* = 6.7 Hz, 1H), 4.04-3.89 (m, 2H), 3.35 (s, 3H), 2.23-2.15 (m, 3H), 1.96-1.91 (m, 2H), 1.87-0.80 (m, 28H), 0.75 (d, *J* = 6.7 Hz, 3H), 0.62 (d, *J*= 6.7 Hz, 3H); ¹³C NMR (50.3 MHz, CDCl₃): δ = 173.7, 137.4, 135.9 (4C), 135.1, 134.9, 134.6, 134.3, 129.4, 129.3, 127.4 (2C), 127.3 (2C), 121.8, 119.2, 117.2, 94.3, 78.5, 71.8, 71.5, 55.7, 40.3, 37.2, 37.0, 35.8, 35.6, 29.7, 28.8, 27.3 (3C), 27.1 (4C), 24.3, 23.8, 21.0 (2C), 19.3; IR (CCl₄): *ν* = 2935, 2905, 2867, 2830, 1728, 1431, 1372, 1159, 1107, 910; [α]_{D}²⁰ = +52.7 (c = 0.77, EtOAc); HRMS (ESI): *m*/*z:* calcd for C₄₃H₆₄NaO₅Si: 711.4421 [*M*+Na]⁺; found: 711.4406.

### Example 36

### 2,2-Dimethyl-propionic acid {(1R,2R)-1-{(1R,2R,6R)-2-[(2R)-2-(tert-butyl-diphenyl-silanoxy)-pent-4-enyl]-6-isopropyl-3-methyl-cyclohex-3-enylmethyl}-2-hydroxy-but-3-enyl} ester.

The compound prepared in Example 35 (32 mg, 0.046 mmol) was dissolved in CH₂Cl₂ (0.5 mL) and cooled to -78 °C. PhSH (10 mg, 0.093 mmol) and BF₃*Et₂O (13 mg, 0.093 mmol) were added and the solution was allowed to warm to -10 °C over 3 h and stirred at -10 °C for 2 h. PhSH (5 mg, 0.046 mmol) and BF₃*Et₂O (7 mg, 0.046 mmol) were added again and the solution stirred at -10 °C for another 1 h. Then a saturated aqueous NaHCO₃ solution (1 mL) was added and the solution was warmed up to room temperature. The layers were separated, the aqueous layer was extracted with EtOAc (3x5 mL) and the combined organic layers were dried over Na₂SO₄. The solvent was evaporated under reduced pressure and the resulting residue was purified by flash chromatography (hexane/EtOAc 9:1) to provide the title compound (19 mg, 63 %) as a colourless oil. R_{f} = 0.40 (hexane/EtOAc 9:1); ¹H NMR (200 MHz, CDCl₃): δ = 7.74-7.63 (m, 4H), 7.48-7.28 (m, 6H), 6.05-5.68 (m, 2H), 5.36-4.88 (m, 6H), 4.08-3.89 (m, 2H), 2.41-2.18 (m, 2H), 2.09-1.95 (m, 1H), 1.84-0.82 (m, 31H), 0.78 (d, J= 6.7 Hz, 3H), 0.66 (d, J= 6.7 Hz, 3H); ¹³C NMR (75.5 MHz, CDCl₃): *δ* = 178.3, 137.3, 137.1, 135.9 (4C), 135.1, 134.6 (2C), 129.5 (2C), 127.4 (4C), 121.7, 117.3, 116.3, 74.3, 73.1, 71.6, 40.5, 37.4, 36.7, 35.6, 35.3, 29.7, 28.7, 27.2 (3C), 27.1 (4C), 24.0, 23.7, 21.0 (2C), 19.3; IR (CCl₄): *ν* = 3580, 3066, 2951, 2922, 2850, 1724, 1425, 1384, 1365, 1152, 1107, 905; [α]_{D}²⁰ = +70.7 (c = 0.81, EtOAc); HRMS (ESI): *m*/*z*: calcd for C₄₁H₆₀NaO₄Si: 667.4159 [M+Na]⁺; found: 667.4128.

### Example 37

### 2,2-Dimethyl-propionic acid [(4R,4aR,6R,7R,11R,12aR)-11-(tert-butyl-diphenyl-silanoxy)-7-hydroxy-4-isopropyl-1-methyl-3,4,4a,5,6,7,10,11,12,12a-decahydro-benzocyclodecen-6-yl] ester.

The compound prepared in Example 36 (18 mg, 0.028 mmol) was dissolved in degassed CH₂Cl₂ (2.3 mL). Grubbs-II (1 mg, 1.4 µmol) in degassed CH₂Cl₂ (0.5 mL) was slowly added. The reaction mixture stirred for 2 d at room temperature. Additional Grubbs-II (1 mg, 1.4 µmol) in degassed CH₂Cl₂ (0.2 mL) was slowly added and the mixture stirred for further 3 d at room temperature. Additional Grubbs-II (1 mg, 1.3 µmol) in degassed CH₂Cl₂ (0.2 mL) was slowly added and the mixture stirred for further 1 d at room temperature. The solvent was evaporated under reduced pressure and the residue was purified by flash chromatography (hexane/EtOAc 9:1) to provide unreacted starting compound (4 mg) and the title compound (10 mg, 55 %, 66 % after recovered starting material) as a colourless oils. R_{*f*} = 0.20 (hexane/EtOAc 9:1); ¹H NMR (400 MHz, CDCl₃): *δ* = 7.66-7.63 (m, 4H), 7.44-7.32 (m, 6H), 6.10-5.91 (m, 2H), 5.75-5.70 (m, 1H), 5.55-5.49 (m, 1H), 5.14-5.05 (m, 2H), 4.40-4.36 (m, 1H), 4.30-3.80 (m, 4H), 2.81-2.73 (m, 1H), 2.38-2.25 (m, 2H), 2.15-2.10 (m, 1H), 1.82-1.10 (m, 16H), 1.06 (s, 9H), 0.85 (d, *J* = 6.8 Hz, 3H), 0.67 (d, *J =* 6.8 Hz, 3H); ¹³C NMR (100.8 MHz, CDCl₃): δ = 173.9, 136.3, 135.9 (2C), 135.8 (2C), 133.9 (2C), 130.3, 129.8, 129.7, 127.7, 127.6 (2C), 127.5 (2C), 119.3, 75.3, 73.1, 72.3, 37.2, 36.5, 35.1, 34.7, 30.5, 27.7, 27.2 (3C), 27.0 (3C), 26.4, 24.4, 22.7, 21.0 (2C), 20.8, 19.2; IR (CCl₄): *ν* = 3618, 3070, 2955, 2923, 2850, 1725, 1477, 1459, 1425, 1384, 1365, 1155, 1100, 1080; [*α*]D²⁰ =+4.0 (c = 0.35, EtOAc); HRMS (ESI): *m*/*z:* calcd for C₃₉H₅₆NaO₄Si: 639.3846 [*M*+Na]⁺; found: 639.3860.

### Example 38

Operating as described in the previous examples, the following compounds are prepared:

### AA 3-Phenyl-acrylic acid 11-acetoxy-1-isopropyl-4-methyl-1,2,4a,5,6,7,10,11,12,12a-decahydro-benzocyclodecen-6-yl ester.

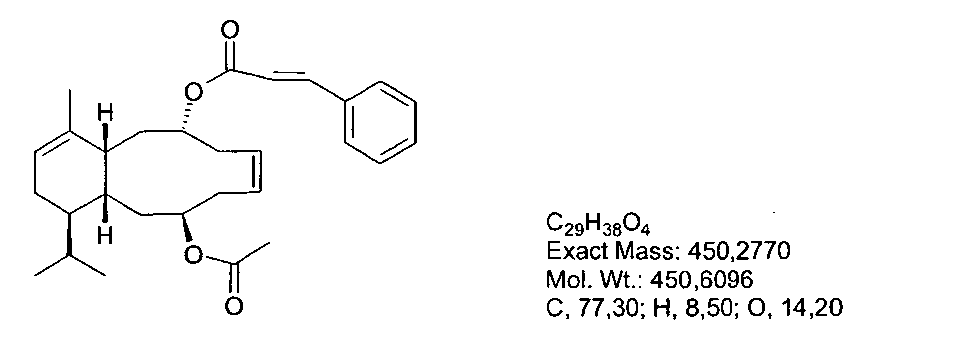

### AB 3-(2-Methyl-thiazol-4-yl)-acrylic acid 11-acetoxy-1-isopropyl-4-methyl-1,2,4a,567,10,11,12,12a-decahydro-benzocyclodecen-6-yl ester.

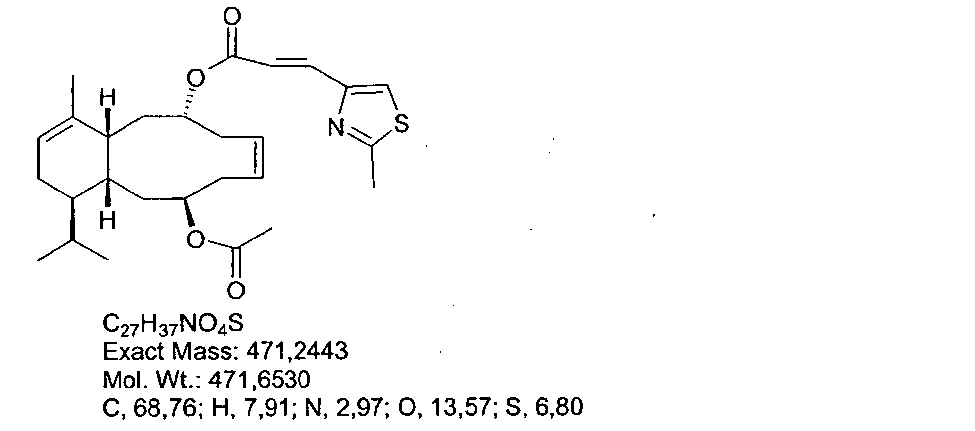

### AC 3-(2-Methyl-oxazol-4-yl)-acrylic acid 11-acetoxy-1-isopropyl-4-methyl-1,2,4a,5,6,7,10,11,12,12a-decahydro-benzocyclodecen-6-yl ester.

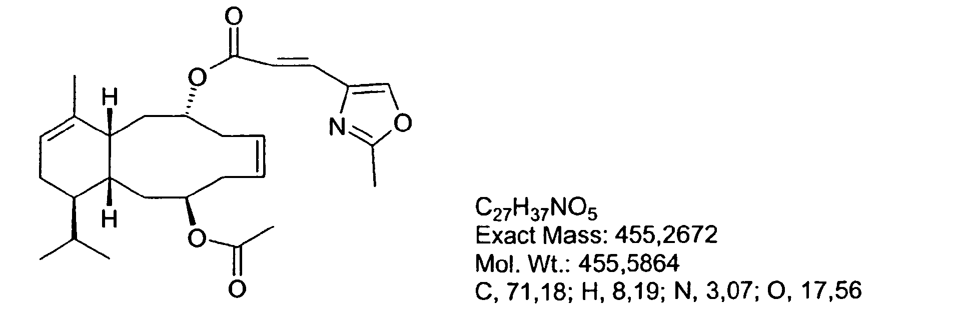

### AD 3-Pyridin-2-yl-acrylic acid 11-acetoxy-1-isopropyl-4-methyl-1,2,4a,5,6,7,10,11,12,12a -decahydro-benzocyclodecen-6-yl ester.

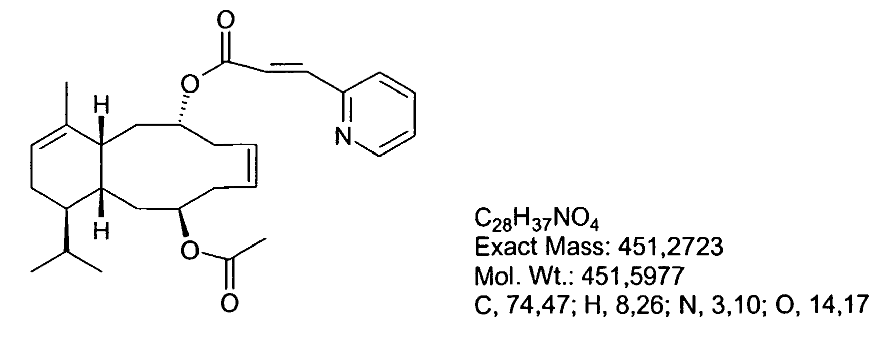

### AE 3-(1-Methyl-1H-imidazol-4-yl)-acrylic acid 11-acetoxy-1-isopropyl-4-methyl-1,2,4a,5,6,7,10,11,12,12a-decahydro-benzocyclodecen-6-yl ester.

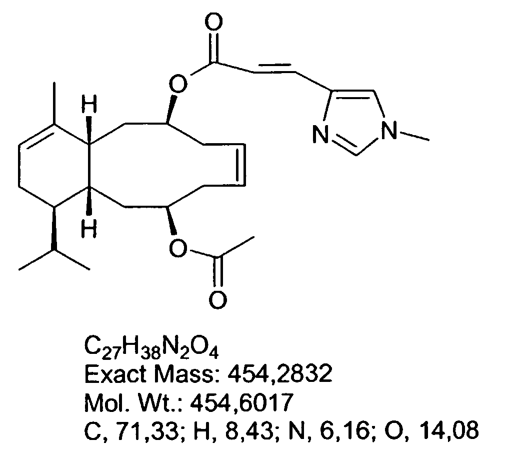

### AF 3-(1-Methyl-1H-imidazol-4-yl)-acrylic acid 11-acetoxy-7-hydroxy-1-isopropyl-4-methyl-1,2,4a,5,6,7,10,11,12,12a-decahydro-benzocyclodecen-6-yl ester.

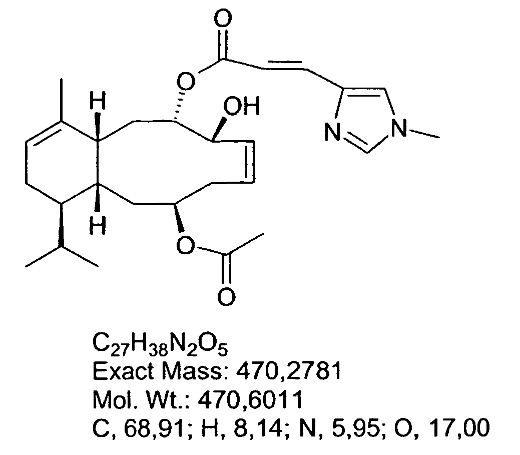

### AG 3-(1-Methyl-1H-imidazol-4-yl)-acrylic acid 11-acetoxy-1-isopropyl-4-methyl-7-oxo-1,2,4a,5,6,7,10,11,12,12a-decahydro-benzocyclodecen-6-yl ester.

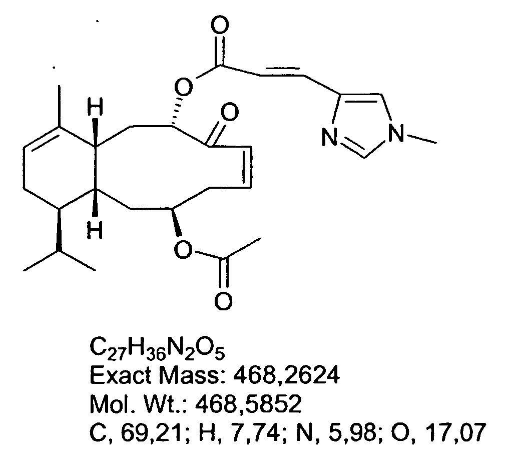

### AH 3-(1-Methyl-1H-imidazol-4-yl)-acrylic acid 7,11-diacetoxy-1-isopropyl-4-methyl-1,2,4a,5,6,7,10,11,12,12a-decahydro-benzocyclodecen-6-yl ester.

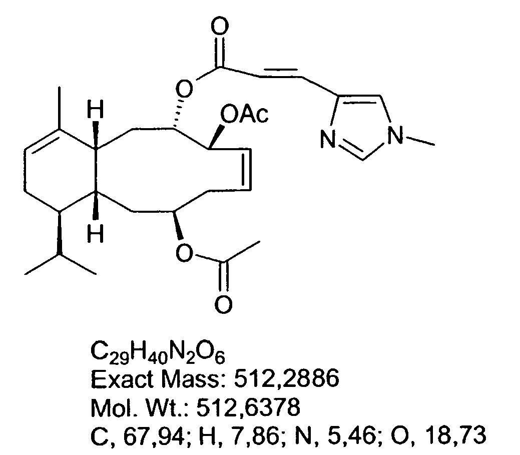

### AI 3-(1-Methyl-1H-imidazol-4-yl)-acrylic acid 11-acetoxy-1-isopropyl-4-methyl-1,2,4a,5,6,7,8,9,10,11,12,12a-dodecahydro-benzocyclodecen-6-yl ester.

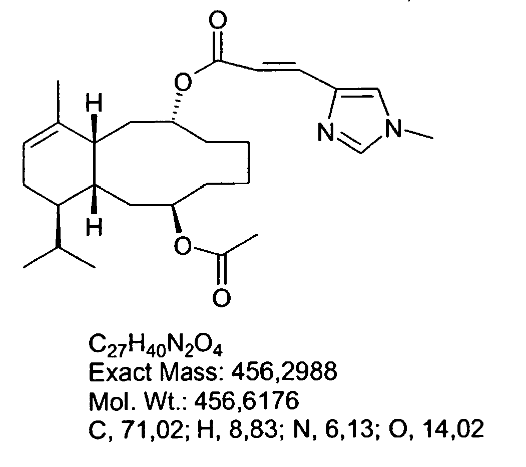

### AL 3-(1-Methyl-1H-imidazol-4-yl)-acrylic acid 9-acetoxy-12-isopropyl-15-methyl-tricyclo[9.4.0.0^{5,7}]pentadec-14-en-3-yl ester.

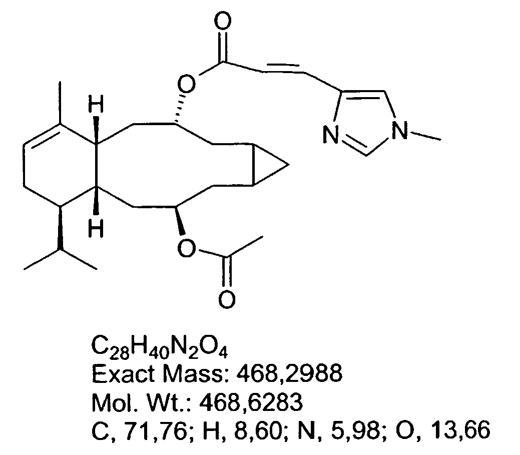

### AM 3-(1-Methyl-1H-imidazol-4-yl)-acrylic acid 11-acetoxy-10-hydroxy-1-isopropyl-4-methyl-1,2,4a,5,6,7,10,11,12,12a-decahydro-benzocyclodecen-6-yl ester.

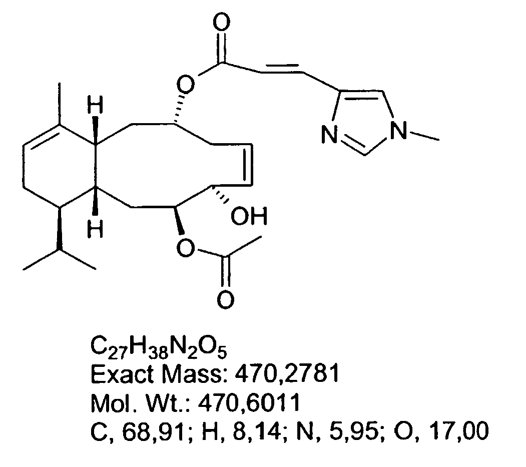

### AN 3-(1-Methyl-1H-imidazol-4-yl)-acrylic acid 10,11-diacetoxy-1-isopropyl-4-methyl-1,2,4a,5,6,7,10,11,12,12a-decahydro-benzocyclodecen-6-yl ester.

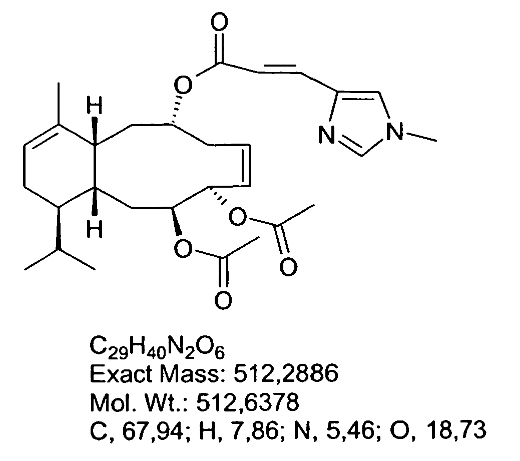

### AO 3-(1-Methyl-1H-imidazol-4-yl)-acrylic acid 11-acetoxy-1-isopropyl-4-methyl-10-oxo-1,2,4a,5,6,7,10,11,12,12a-decahydro-benzocyclodecen-6-yl ester.

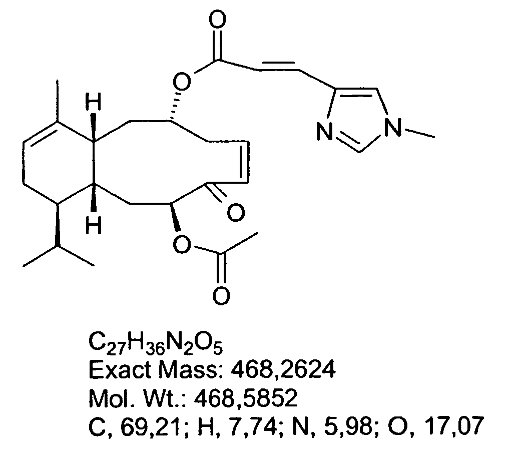

### AP 3-(1-Methyl-1H-imidazol-4-yl)-acrylic acid 11-acetoxy-1-isopropyl-4-methyl-10-oxo-1,2,4a,5,6,7,10,11,12,12a-decahydro-benzocyclodecen-6-yl ester.

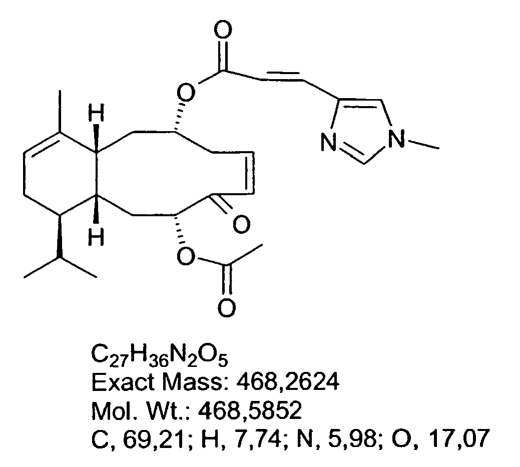

### AQ 3-(1-Methyl-1H-imidazol-4-yl)-acrylic acid 11-acetoxy-10-hydroxy-1-isopropyl-4-methyl-1,2,4a,5,6,7,10,11,12,12a-decahydro-benzocyclodecen-6-yl ester

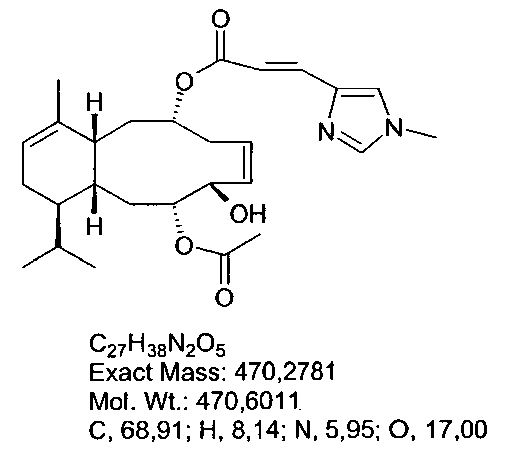

### AR 3(1-Methyl-1H-imidazol-4-yl)-acrylic acid 10,11-diacetoxy-1-isopropyl-4-methyl-1,2,4a,5,6,7,10,11,12,12a-decahydro-benzocyctodecen-6-yl ester.

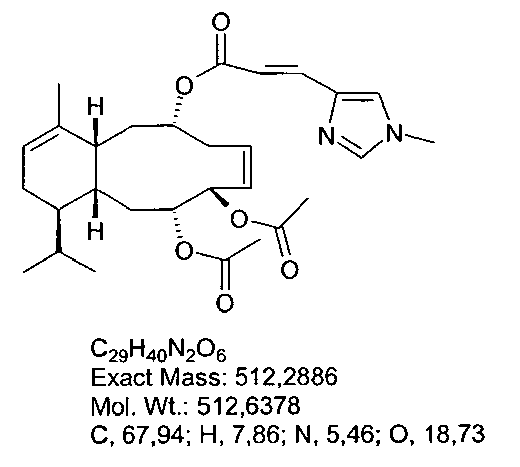

### AS 3-(1-Methyl-1H-imidazol-4-yl)-acrylic acid 11-hydroxy-1-isopropyl-4-methyl-1,2,4a,5,6,7,10,11,12,12a-decahydro-benzocyclodecen-6-yl ester.

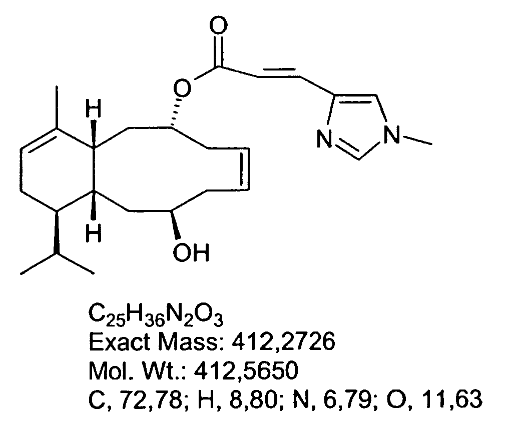

### AT 3-(1-Methyl-1H-imidazol-4-yl)-acrylic acid 11-acetoxy-1-isopropyl-4-methyl-1,2,4a,5,6,7,10.11,12,12a-decahydro-benzocyclodecen-6-yl ester

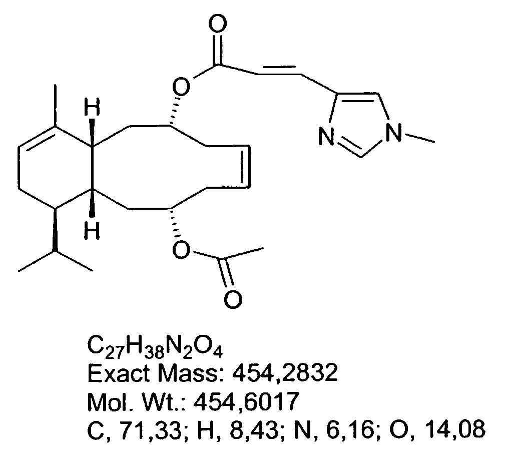

### AU 3-(1-Methyl-1H-imidazol-4-yl)-acrylic acid 11-hydroxy-1-isopropyl-4-methyl-1,2,4a,5,6,7,10,11,12,12a-decahydro-benzocyclodecen-6-yl ester.

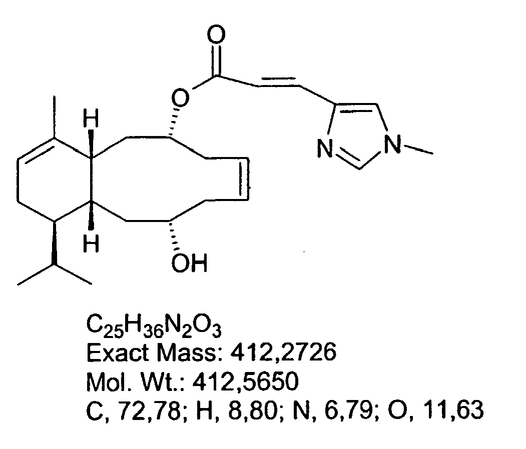

### AV (E)-3-(1-Methyl-1H-imidazol-4-yl)-acrylic acid [(1R,4aR,6R,10R,11R,12aR)-11-(2,2-dimethyl-propionyloxy)-1-isopropyl-10-methoxy-4-methyl-1,2,4a,5,6,7,10,11,12,12a-decahydro-benzocyclodecen-6-yl] ester.

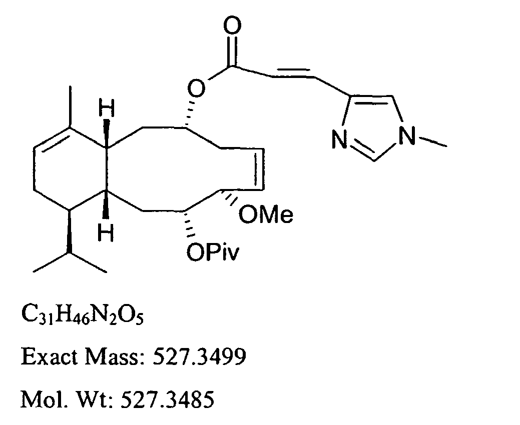

### AX (E)-3-(1-Methyl-1H-imidazol-4-yl)-acrylic acid [(1R, 4aR,6R,10R,11R,12aR)-11-(2,2-dimethyl-propionyloxy-1-isopropyl-10-hydroxy-4-methyl-1,2,4a,5,6,7,10,11,12,12a-decahydro-benzocyclodecen-6-yl] ester.

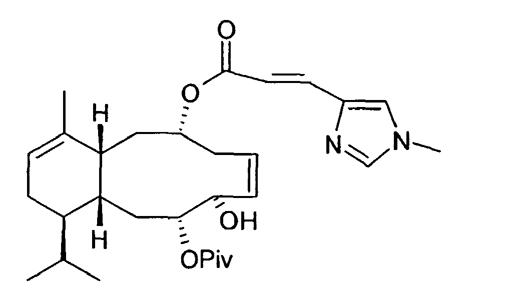

### AY 3-(1-Methyl-1H-imidazol-4-yl)-acrylic acid 1-isopropyl-11-methoxycarbonylmethylene-4-methyl-1,2,4a,5,6,7,10,11,12,12a-decahydro-benzocyclodecen-6-yl ester.

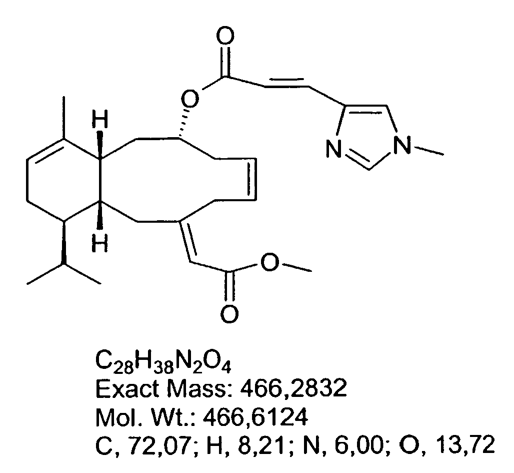

### AW 3-(1-Methyl-1H-imidazol-4-yl)-acrylic acid 1-isopropyl-11-methoxymethylene-4-methyl-1,2,4a,5,6,7,10,11,12,12a-decahydro-benzocyclodecen-6-yl ester.

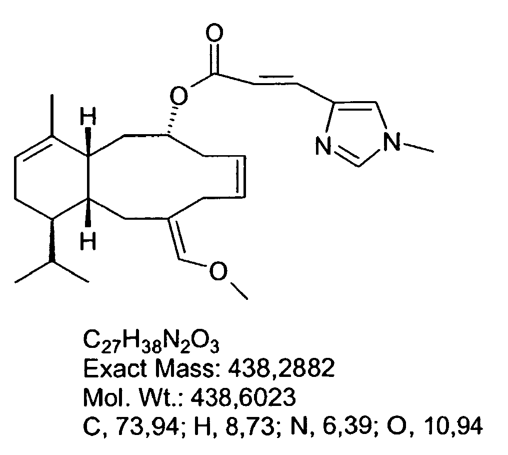

### AZ 3-(1-Methyl-1H-imidazol-4-yl)-acrylic acid 11-ethoxycarbonylmethylene-1-isopropyl-4-methyl-1,2,4a,5,6,7,10,11,12,12a-decahydro-benzocyclodecen-6-yl ester.

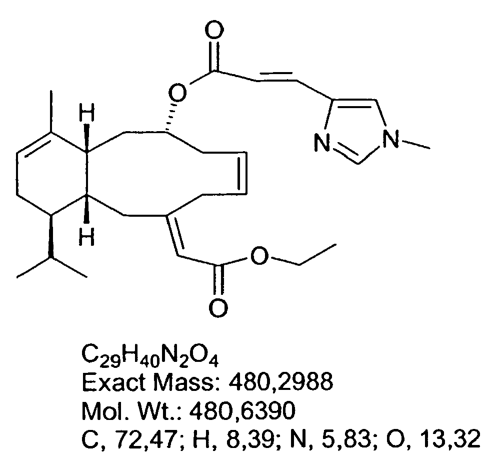

### BA 3-(1-Methyl-1H-imidazol-4-yl)-acrylic acid 11-formyl-1-isopropyl-4-methyl-1,2,4a,5,6,7,10,11,12,12a-decahydro-benzocyclodecen-6-yl ester.

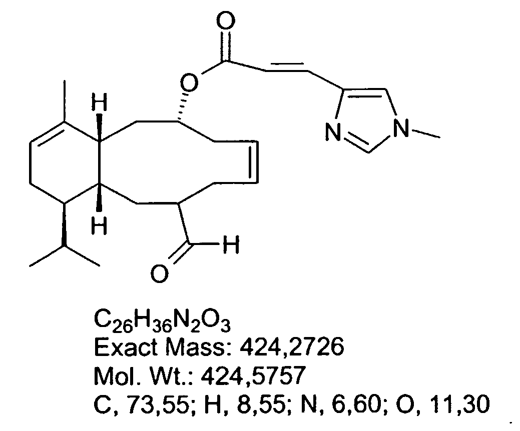

### BB 3-(1-Methyl-1H-imidazol-4-yl)-acrylic acid 11-hydroxymethyl-1-isopropyl-4-methyl-1,2,4a,5,6,7,10,11,12,12a-decahydro-benzocyclodecen-6-yl ester.

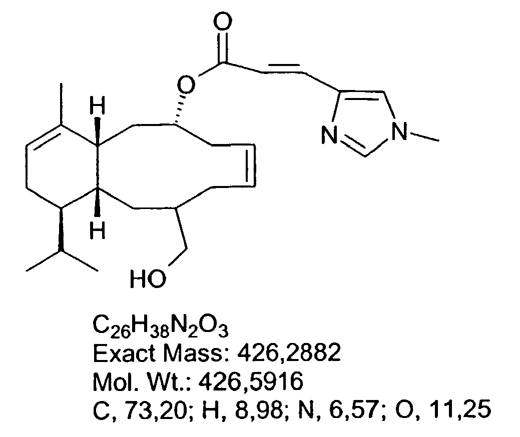

### BC 3-(1-Methyl-1H-imidazol-4-yl)-acrylic acid 11-(3-acetoxy-4,5-dihydroxy-tetrahydropyran-2-yloxymethyl)-1-isopropyl-4-methyl-1,2,4a,5,6,7,10,11,12,12a-decahydro-benzocyclodecen-6-yl ester.

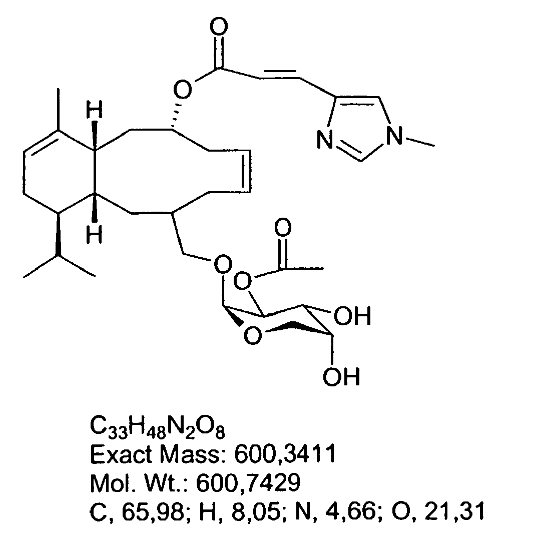

### BD 4-Isopropyl-1-methyl-11-[3-(1-methyl-1H-imidazol-4-yl)-acryloyloxyl-3,4,4a,5,6,7,10,11,12,12a-decahydro-benzocyclodecene-6-carboxylic acid ethyl ester.

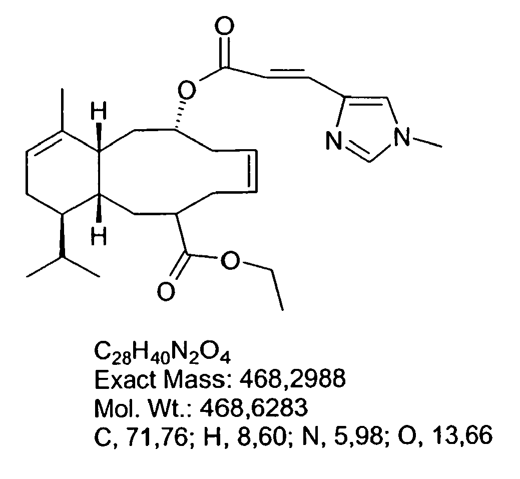

### BE 7-Hydroxy-4-isopropyl-1-methyl-11-[3-(1-methyl-1H-imidazol-4-yl)-acryloyloxyl-3,4,4a,7,10,11,12,12a-octahydro-benzocyclodecene-6-carboxylic acid ethyl ester.

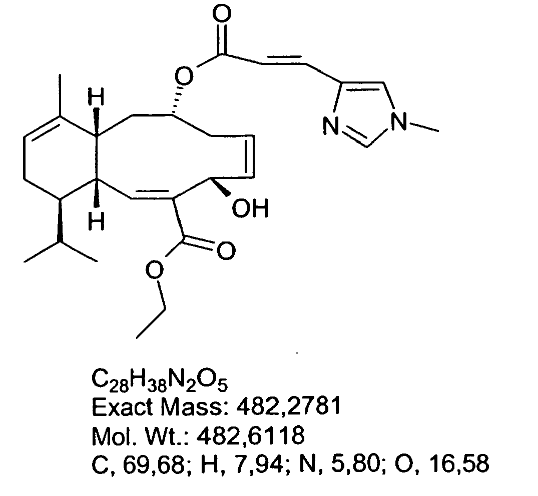

### BF 7-Hydyroxy-4-isopropyl-1-methyl-11[3-(1-methyl-1H-imidazol-4-yl)-acryloyloxy]-3,4,4a,7,10,11,12,12a-octahydro-benzocyclodecene-6-carboxylic acid ethyl ester.

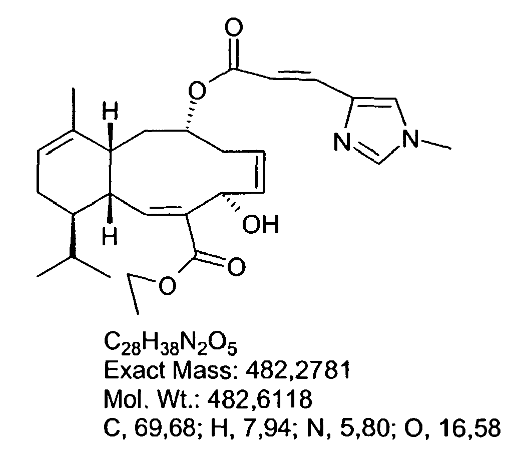

### BG (E)-3-(1-Methyl-1H-imidazol-4-yl)-acrylic acid [(1R,4aR,6R,11R,12aR)-11-(2,2-dimethyl-propionyloxy)-1-isopropyl-10-oxo-4-methyl-1,2,4a,5,6,7,10,11,12,12a-decahydro-benzocyclodecen-6-yl] ester.

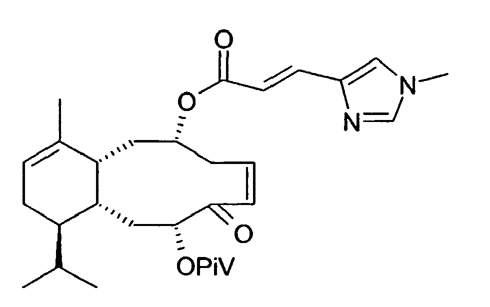

### BH (E)-3-(1-Methyl-1H-imidazol-4 yl)-acrylic acid [(1R,4aR,6R,10 R,11R,12aR)-11-(2,2-dimethyl-propionyloxy)-1-isopropyl-10-acetoxy-4-methyl-1,2,4a,5,6,7,10,11,12,12a-decahydro-benzocyclodecen-6-yl] ester.

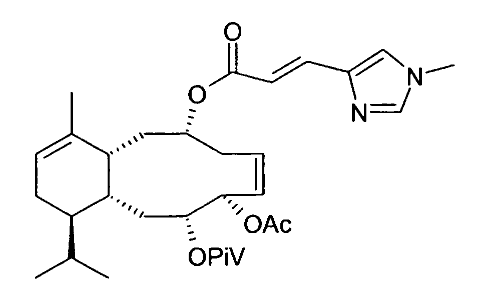

## Claims

1. A compound which is a sarcodictyin derivative having the formula I: 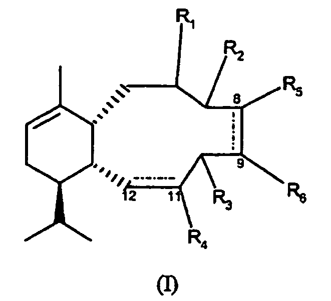 wherein:
----- at positions 8-9 and 11-12 independently represents a single or double bond,
-R₁ represents oxygen (=O), or a residue -OR₇, wherein R₇ represents hydrogen, linear or branched C₁-C₇ alkanoyl, benzoyl, C₁-C₁₀ alkyl, C₂-C₁₀ alkenyl or a residue of the formula 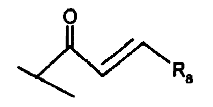
wherein R₈ is an aryl or heterocyclyl;
one of -R₂ and -R₃ represents hydrogen and the other one is chosen from the group consisting of hydrogen, oxygen (=O) and a residue -OR₉, wherein R₉ represents hydrogen, C₁-C₆ alkanoyl or benzoyl;
when ---- at position 11-12 represents a single bond, then -R₄ represents oxygen (=O), methylene (=CH₂), =CHCOOR₁₀, wherein R₁₀ represents C₁-C₁₀ alkyl or aryl; =CH(OCH₃), or a residue of formula
-OR₉, wherein R₉ is as defined above;
-CH₂OR₁₁ wherein R₁₁ represents hydrogen or a sugar residue, or
-COR₁₂ wherein R₁₂ represents hydrogen, -OH or -OR₁₀, wherein R₁₀ is as defined above; or
when ----- at position 11-12 represents a double bond, then -R₄ represents a residue of formula -CH₂OR₁₁ or -COR₁₂ as defined above; - R₅ and -R₆ are both hydrogen or, when =
---- at position 8-9 represents a single bond, taken together with the carbon atoms to which they are attached form a cyclopropane ring; or a pharmaceutically acceptable salt thereof.

2. A compound according to claim I wherein the sarcodictyin derivative is of formula Ia 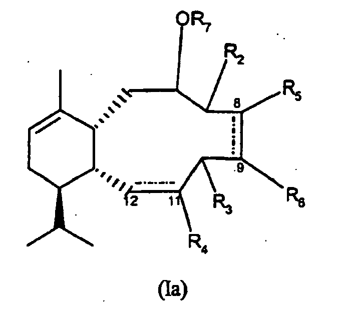 wherein:
----- at positions 8-9 and 11-12 independently represents a single or double bond,
R₇ represents a residue of the formula 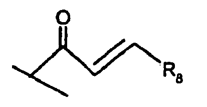
wherein R₈ is N-methyl imidazolyl, phenyl, methyl-thiazolyl, methyl-oxazolyl or pyridyl group;
one of -R₂ and -R₃ represents hydrogen and the other one is hydrogen or oxygen (=O), hydroxy or acetoxy-,
when ----- at position 11-12 represents a single bond, then -R₄ represents oxygen (=O), methylene (=CH₂), =CHCOOR₁₀, wherein R₁₀ represents methyl or ethyl, =CH(OCH₃), -CHO; hydroxy, acetoxy, pivaloyloxy or -CH₂OR₁₁ wherein R₁₁ represents hydrogen or a sugar residue having the formula: 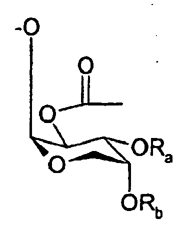
wherein Rₐ and R_{b} independently represent hydrogen, a hydroxy protecting group, or C₁-C₆ alkanoyl,
or, when ----- at position 11-12 represents a double bond, then -R₄ represents a residue of formula -CO₂C₂H₅; and
-R₅ and -R₆ are both hydrogen or, when ----- at position 8-9 represents a single bond, taken together with the carbon atoms to which they are attached form a cyclopropane ring.

3. A compound as claimed in claim 2 wherein the substituent OR₇ in formula Ia is under the plane and the substituent R₄ is above the plane; R₂ and R₃ are hydrogen and R₈ is N-methyl imidazolyl.

4. A process for producing a compound as defined in claim 1, which process comprises cyclizing a compound of formula II 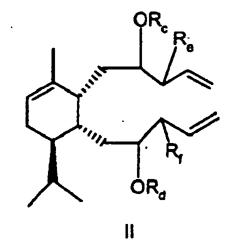 wherein R_{c} represents hydrogen, a silyl protecting group, C₁-C₆ alkanoyl or benzoyl or, taken together with Rₑ, forms an acetonide ring; R_{d} represents hydrogen,
C₁-C₆ alkanoyl, or benzoyl, or , taken together with R_{f}, forms an acetonide ring; either Rₑ represents H and R_{f} either represents OH or is linked to the adjacent OR_{d} substituent as defined above, or R_{f} represents H and Rₑ either represents OH or is linked to the adjacent OR_{c} substituent as defined above;
and, if desired, converting one resulting sarcodictyin derivative of formula I', 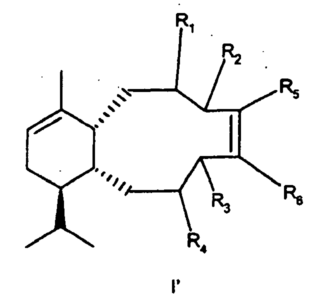 wherein R₁ is OR_{c}, R₂ is Rₑ, R₃ is R_{f}, R₄ is OR_{d}, in which R_{c}, R_{d}, Rₑ and R_{f} are as defined above and R₅ and R₆ are hydrogen, into another sarcodictyin derivative of formula I as defined in claim 1 and/or, if desired, converting a sarcodictyin derivative of formula I' or I into a pharmaceutically acceptable salt thereof; and/or, if desired converting a pharmaceutically acceptable salt of a sarcodictyin derivative of formula I or I' into the corresponding free compound.

5. A process according to claim 4 wherein the cyclization is carried out through the Ring Closing Metathesis (RCM) reaction.

6. A process according to claim 5 in which the RCM reaction is carried out in the presence of a Nolan and Grubb's catalyst

7. A pharmaceutical composition which comprises a pharmaceutically acceptable diluent or carrier and, as an active ingredient, a compound as defined in any of claims 1 to 3.

8. A compound as defined in any one of claims 1 to 3 for use in a method of treatment of the human or animal body by therapy or prophylaxis.

9. A compound as claimed in claim 8 for use as an antitumour agent.

10. Use of a compound as defined in any one of claims 1 to 3 in the manufacture of a medicament for use as an antitumour agent.

11. Use of a compound as defined in any one of claims 1 to 3 in the manufacture of a medicament for use in treating leukemia or a solid tumour.

## Patentansprüche

1. Eine Verbindung, die ein Sarcodictyinderivat ist, welches die Formel I hat: 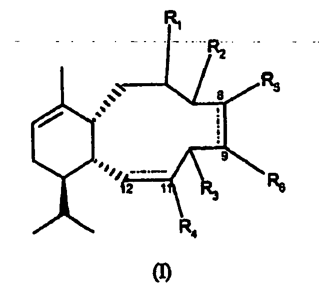 worin:
---- an Positionen 8-.9 und 11-12 unabhängig eine Einzel- oder Doppelbindung darstellt, -R₁ stellt Sauerstoff (=O) oder einen Rest -OR₇ dar, worin R₇ Wasserstoff darstellt, gerades oder verzweigtes C₁-C₇ Alkanoyl, Benzoyl, C₁-C₁₀ Alkyl, C₂-C₁₀ Alkenyl oder einen Rest mit der Formel 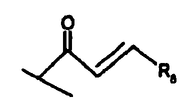
worin R₈ ein Aryl oder Heterocyclyl ist;
eines von -R₂ und -R₃ stellt Wasserstoff dar und das andere ist gewählt aus der Gruppe bestehend aus Wasserstoff, Sauerstoff (=O) und einem Rest -OR₉, worin R₉ Wasserstoff, C₁-C₆ Alkanoyl oder Benzoyl darstellt;
wenn ---- an Position 11-12 eine Einzelbindung darstellt, dann stellt -R₄ Sauerstoff (=O), Methylen (=CH₂), =CHCOOR₁₀ dar, worin R₁₀ C₁-C₁₀ Alkyl oder Aryl darstellt; =CH(OCH₃) oder einen Rest der Formel -OR₉, worin R₉ wie oben definiert ist;
-CH₂OR₁₁, worin R₁₁ Wasserstoff oder einen Zuckerrest darstellt oder
-COR₁₂, worin R₁₂ Wasserstoff, -OH oder -OR₁₀ darstellt, worin R₁₀ wie oben definiert ist;
oder
wenn ---- an Position 11-12 eine Doppelbindung darstellt, dann stellt -R₄ einen Rest der Formel -CH₂OR₁₁ oder -COR₁₂ dar, wie oben definiert; -R₅ und -R₆ sind beide Wasserstoff oder bilden, wenn
---- an Position 8-9 eine Einzelbindung darstellt, zusammengenommen mit den Kohlenstoffatomen, an welche sie gebunden sind, einen Cyclopropanring; oder ein pharmazeutisch verträgliches Salz davon.

2. Eine Verbindung gemäß Anspruch I, worin das Sarcodictyinderivat die Formel Ia hat 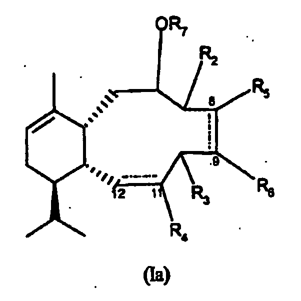 worin:
---- an Positionen 8-9 und 11-12 unabhängig eine Einzel- oder Doppelbindung darstellt,
R₇ stellt einen Rest der folgenden Formel dar 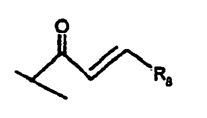
worin R₈ N-Methylimidazolyl, Phenyl, Methyl-Thiazolyl, Methyl-Oxazolyl oder eine Pyridylgruppe ist;
eins von -R₂ und -R₃ stellt Wasserstoff dar und das andere ist Wasserstoff oder Sauerstoff (=O), Hydroxy oder Acetoxy;
wenn ---- an Position 11-12 eine Einzelbindung darstellt, dann stellt -R₄ Sauerstoff (=O), Methylen (=CH₂), =CHCOOR₁₀ dar, worin R₁₀ Methyl oder Ethyl, =CH(OCH₃), -CHO darstellt; Hydroxy, Acetoxy, Pivaloyloxy oder -CH₂OR₁₁, worin R₁₁ Wasserstoff oder einen Zuckerrest der folgenden Formel darstellt: 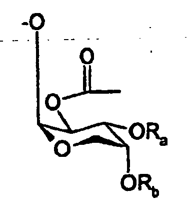
worin Rₐ und R_{b} unabhängig Wasserstoff, eine Hydroxyschutzgruppe oder C₁-C₆ Alkanoyl darstellen,
oder wenn ---- an Position 11-12 eine Doppelbindung darstellt, dann stellt -R₄ einen Rest der Formel -CO₂C₂H₅ dar; und
-R₅ und -R₆ sind beide Wasserstoff oder bilden, wenn ---- an Position 8-9 eine Einzelbindung darstellt, zusammengenommen mit den Kohlenstoffatomen, an welche sie gebunden sind, einen Cyclopropanring.

3. Eine Verbindung wie in Anspruch 2 beansprucht, worin der Substituent OR₇ in Formel Ia unter der Ebene ist, und der Substituent R₄ über der Ebene ist; R₂ und R₃ sind Wasserstoff und R₈ ist N-Methylimidazolyl.

4. Ein Verfahren zur Herstellung einer Verbindung wie in Anspruch 1 definiert, wobei das Verfahren Zyklisieren einer Verbindung der Formel II umfaßt: 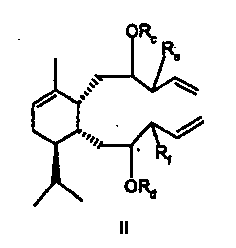 worin R_{c} Wasserstoff, eine Silylschutzgruppe, C₁-C₆ Alkanoyl oder Benzoyl darstellt, oder zusammengenommen mit Rₑ einen Acetonidring bildet; R_{d} stellt Wasserstoff, C₁-C₆ Alkanoyl oder Benzyol dar, oder bildet zusammengenommen mit R_{f} einen Acetonidring; entweder stellt Rₑ H dar und R_{f} stellt entweder OH dar oder ist gebunden an den angrenzenden OR_{d} Substituenten, wie oben definiert, oder R_{f} stellt H dar und Rₑ stellt entweder OH dar oder ist gebunden an den angrenzenden OR_{c} Substituenten, wie oben definiert;
und, wenn gewünscht, Umwandeln eines resultierenden Sarcodictyinderivats der Formel I' 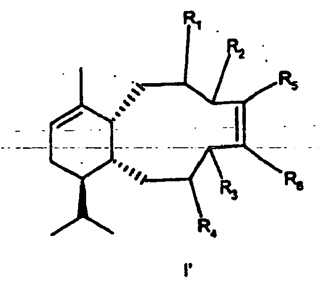 worin R₁ OR_{c} ist, R₂ Rₑ ist, R₃ R_{f} ist, R₄ OR_{d} ist, in welchen R_{c}, R_{d}, Rₑ und R_{f} wie oben definiert sind und R₅ und R₆ sind Wasserstoff, in ein anderes Sarcodictyinderivat der Formel I, wie in Anspruch 1 definiert, und/oder, wenn gewünscht, Umwandeln eines Sarcodictyinderivats der Formel I' oder I in ein pharmazeutisch verträgliches Salz davon; und/oder, wenn gewünscht, Umwandeln eines pharmazeutisch verträglichen Salzes eines Sarcodictyinderivats der Formel I oder I' in die entsprechende freie Verbindung.

5. Ein Verfahren gemäß Anspruch 4, worin die Zyklisierung durch die Ring-schließende Metathese- (Ring Closing Metathesis, RCM) Reaktion durchgeführt wird.

6. Ein Verfahren gemäß Anspruch 5, worin die RCM Reaktion in der Anwesenheit eines Nolan und Grubb's Katalysators durchgeführt wird.

7. Eine pharmazeutische Zusammensetzung, welche ein pharmazeutisch verträgliches Verdünnungsmittel oder Träger, und als einen aktiven Bestandteil, eine Verbindung wie in irgendeinem der Ansprüche 1 bis 3 definiert, umfaßt .

8. Eine Verbindung wie in irgendeinem der Ansprüche 1 bis 3 definiert, für die Verwendung in einem Verfahren zur Behandlung des menschlichen oder tierischen Körpers durch Therapie oder Prophylaxe.

9. Eine Verbindung wie in Anspruch 8 beansprucht, für die Verwendung als ein anti-Tumorwirkstoff.

10. Verwendung einer Verbindung wie in irgendeinem der Ansprüche 1 bis 3 definiert, in der Herstellung eines Medikaments für die Verwendung als ein anti-Tumorwirkstoff.

11. Verwendung einer Verbindung wie in irgendeinem der Ansprüche 1 bis 3 definiert, in der Herstellung eines Medikaments für die Verwendung in der Behandlung von Leukämie oder einem festen Tumor.

## Revendications

1. Composé qui est un dérivé de sarcodictyine répondant à la formule I : 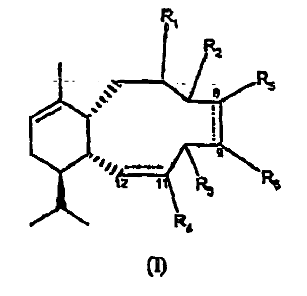 dans laquelle :
----- en positions 8-9 et 11-12 représentent indépendamment une liaison simple ou une double liaison,
-R₁ représente un atome d'oxygène (=O) ou un résidu -OR₇, dans lequel R₇ représente un atome d'hydrogène, un groupe alcanoyle en C₁ à C₇ linéaire ou ramifié, benzoyle, alkyle en C₁ à C₁₀, alcényle en C₂ à C₁₀ ou un résidu de formule 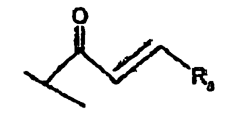
dans laquelle R₈ représente un groupe aryle ou hétérocyclyle ;
un des groupes -R₂ et -R₃ représente un atome d'hydrogène et l'autre est choisi dans le groupe consistant en un atome d'hydrogène, un atome d'oxygène (=O) et un résidu -OR₉, dans lequel R₉ représente un atome d'hydrogène, un groupe alcanoyle en C₁ à C₆ ou benzoyle ;
lorsque ----- en position 11-12 représente une liaison simple, alors -R₄ représente un atome d'oxygène (=O), un groupe méthylène (=CH₂), un groupe =CHCOOR₁₀ dans lequel R₁₀ représente un groupe alkyle en C₁ à C₁₀ ou aryle ; un groupe =CH(OCH₃), ou un résidu de formule -OR₉, dans laquelle R₉ répond à la définition précitée ;
un groupe -CH₂OR₁₁ dans lequel R₁₁ représente un atome d'hydrogène ou un résidu d'un sucre, ou
un groupe -COR₁₂ dans lequel R₁₂ représente un atome d'hydrogène, un groupe -OH ou -OR₁₀ dans lequel R₁₀ répond à la définition précitée ;
ou
lorsque ----- en position 11-12 représente une double liaison, alors -R₄ représente un résidu de formule -CH₂OR₁₁ ou -COR₁₂ répondant à la définition précitée ; -R₅ et -R₆ représentent l'un et l'autre un atome d'hydrogène
ou, lorsque ----- en position 8-9 représente une liaison simple, ils sont pris conjointement avec les atomes de carbone auxquels ils sont fixés pour former un noyau cyclopropane ; ou un de ses sels pharmaceutiquement acceptables.

2. Composé suivant la revendication 1, dans lequel le dérivé de sarcodictyine répond à la formule Ia 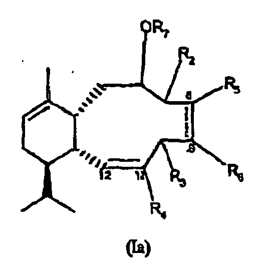 dans laquelle :
---- en positions 8-9 et 11-12 représentent indépendamment une liaison simple ou une double liaison,
R₇ représente un résidu de formule 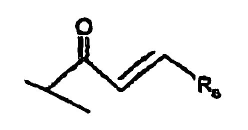
dans laquelle
R₈ représente un groupe N-méthylimidazolyle, phényle, méthylthiazolyle, méthyloxazolyle ou pyridyle ;
un de -R₂ et -R₃ représente un atome d'hydrogène et l'autre représente un atome d'hydrogène ou un atome d'oxygène (=O), un groupe hydroxy ou acétoxy ;
lorsque ----- en position 11-12 représente une liaison simple, alors -R₄ représente un atome d'oxygène (=O), un groupe méthylène (=CH₂), un groupe =CHCOOR₁₀ dans lequel R₁₀ représente un groupe méthyle ou éthyle, =CH(OCH₃), -CHO ; hydroxy, acétoxy, pivaloyloxy ou -CH₂R₁₁ dans lequel R₁₁ représente un atome d'hydrogène ou un résidu d'un sucre de formule : 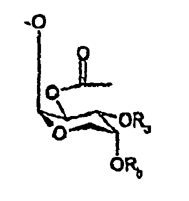 dans laquelle Rₐ et R_{b} représentent indépendamment un atome d'hydrogène, un groupe protecteur de la fonction hydroxy ou un groupe alcanoyle en C₁ à C₆,
ou, lorsque ---- en position 11-12 représente une double liaison, alors -R₄ représente un résidu de formule -CO₂C₂H₅ ; et
-R₅ et -R₆ représentent l'un et l'autre un atome d'hydrogène ou, lorsque ---- en position 8-9 représente une liaison simple, ils sont pris conjointement avec les atomes de carbone auxquels ils sont fixés pour former un noyau cyclopropane.

3. Composé suivant la revendication 2, dans lequel le substituant OR₇, dans la formule Ia, est au-dessous du plan et le substituant R₄ est au-dessus du plan ; R₂ et R₃ représentent des atomes d'hydrogène et R₈ représente un groupe N-méthylimidazolyle.

4. Procédé pour la production d'un composé tel que défini dans la revendication 1, procédé qui comprend la cyclisation d'un composé de formule II 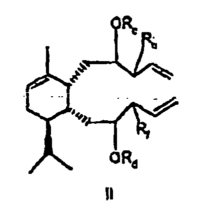 dans laquelle R_{c} représente un atome d'hydrogène, un groupe protecteur silyle, un groupe alcanoyle en C₁ à C₆ ou benzoyle ou bien, pris conjointement avec R_{c}, forme un noyau acétonide ; R_{d} représente un atome d'hydrogène,
un groupe alcanoyle en C₁ à C₆ ou benzoyle ou bien, pris conjointement avec R_{f}, forme un noyau acétonide ; Rₑ représente H et R_{f} représente un groupe OH ou est lié au substituant OR_{d} adjacent comme défini ci-dessous, ou bien R_{f} représente H et Rₑ représente un groupe OH ou est lié au substituant OR_{c} adjacent comme défini ci-dessus ;
et, si cela est désiré, la conversion d'un dérivé de sarcodictyine résultant de formule I', 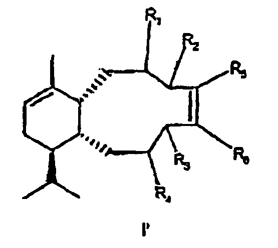 dans laquelle R₁ représente un groupe OR_{c}, R₂ représente un groupe Rₑ, R₃ représente un groupe R_{f}, R₄ représente un groupe OR_{d}, dans lesquels R_{c}, R_{d}, Rₑ et R_{f} répondent aux définitions précitées, et R₅ et R₆ représentent des atomes d'hydrogène, en un autre dérivé de sarcodictyine de formule I tel que défini dans la revendication 1 et/ou, si cela est désiré, la conversion d'un dérivé de sarcodictyine de formule I' ou I en un de ses sels pharmaceutiquement acceptables ; et/ou, si cela est désiré, la conversion d'un sel pharmaceutiquement acceptable d'un dérivé de sarcodictyine de formule I ou I' en le composé libre correspondant.

5. Procédé suivant la revendication 4, dans lequel la cyclisation est effectuée par une réaction de double décomposition avec cyclisation (RCM).

6. Procédé suivant la revendication 5, dans laquelle la réaction RCM est effectuée en présence d'un catalyseur de Nolan et Grubb.

7. Composition pharmaceutique qui comprend un diluant ou support pharmaceutiquement acceptable et, comme ingrédient actif, un composé tel que défini dans l'une quelconque des revendications 1 à 3.

8. Composé tel que défini dans l'une quelconque des revendications 1 à 3, destiné à être utilisé dans une méthode de traitement de l'organisme de l'homme ou d'un animal, par thérapie ou prophylaxie.

9. Composé suivant la revendication 8, destiné à être utilisé comme agent antitumoral.

10. Utilisation d'un composé tel que défini dans l'une quelconque des revendications 1 à 3, dans la production d'un médicament destiné à être utilisé comme agent antitumoral.

11. Utilisation d'un composé tel que défini dans l'une quelconque des revendications 1 à 3, dans la production d'un médicament destiné à être utilisé dans le traitement de la leucémie ou d'une tumeur solide.
